(19) Europäisches Patentamt
European Patent Office
Office européen des brevets

(11) EP 3 249 384 B1

(12) **EUROPEAN PATENT SPECIFICATION**

(45) Date of publication and mention of the grant of the patent:
**09.09.2020 Bulletin 2020/37**

(51) Int Cl.:
***G01N 15/14** (2006.01)* ***G01N 1/30** (2006.01)*
***G01N 21/64** (2006.01)* *C12M 1/00 (2006.01)*
*G01N 15/00 (2006.01)*

(21) Application number: **17173030.2**

(22) Date of filing: **26.05.2017**

(54) **SPECIMEN ANALYZING METHOD AND SPECIMEN ANALYZER**

PROBENANALYSEVERFAHREN UND PROBENANALYSATOR

PROCÉDÉ D’ANALYSE D’ÉCHANTILLONS ET ANALYSEUR D’ÉCHANTILLONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.05.2016 JP 2016105610**

(43) Date of publication of application:
**29.11.2017 Bulletin 2017/48**

(73) Proprietor: **SYSMEX CORPORATION**
**Kobe-shi**
**Hyogo 651-0073 (JP)**

(72) Inventors:
- **Kono, Mari**
  **Hyogo, 651-0073 (JP)**
- **Takagi, Yuri**
  **Hyogo, 651-0073 (JP)**
- **Kishimoto, Shouhei**
  **Hyogo, 651-0073 (JP)**

(74) Representative: **Hoffmann Eitle**
**Patent- und Rechtsanwälte PartmbB**
**Arabellastraße 30**
**81925 München (DE)**

(56) References cited:
**EP-A2- 2 587 262**

- **WALID M. AL-GHOUL ET AL: "Evidence for simvastatin anti-inflammatory actions based on quantitative analyses of NETosis and other inflammation/oxidation markers", RESULTS IN IMMUNOLOGY, vol. 4, 25 March 2014 (2014-03-25), pages 14-22, XP055413479, ISSN: 2211-2839, DOI: 10.1016/j.rinim.2014.03.001**
- **VOLKER BRINKMANN ET AL: "Automatic quantification of in vitro NET formation", FRONTIERS IN IMMUNOLOGY, vol. 3, 1 January 2013 (2013-01-01), XP055367847, DOI: 10.3389/fimmu.2012.00413**
- **ZHAO WENPU ET AL: "A novel image-based quantitative method for the characterization of NETosis", JOURNAL OF IMMUNOLOGICAL METHODS, vol. 423, 20 May 2015 (2015-05-20), pages 104-110, XP029247232, ISSN: 0022-1759, DOI: 10.1016/J.JIM.2015.04.027**
- **YURI TAKAGI ET AL: "Comparison of optical data from flow cytometry and microscopy of leukocytes after exposure to specific reagents", MICROSCOPY, vol. 64, no. 5, 9 October 2015 (2015-10-09), pages 305-310, XP055413984, ISSN: 2050-5698, DOI: 10.1093/jmicro/dfv023**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The invention relates to a specimen analyzing method and a specimen analyzer.

BACKGROUND

Neutrophil extracellular traps are net-like structures released from neutrophils in order to eliminate or kill pathogens in blood or tissues. The neutrophil extracellular traps contain granule proteins and chromatin fibers. As a method for detecting neutrophil extracellular traps, a method using flow cytometry is described in Non-Patent Document 1 (Walid M. Al-Ghoul, et al., Evidence for simvastatin anti-inflammatory actions based on quantitative analyses of NETosis and other inflammation/oxidation markers, Results in Immunology, Vol. 4, 2014, pp. 14-22).
Brinkmann et al. (Front Immunol 3: 413, 2013) decribe automatic quantification of in vitro NET formation. EP 2 587 262 A2 relates to a detection method of activated neutrophils.

SUMMARY

The inventors, however, have found that the technique described in Non-Patent Document 1 has low sensitivity for separating neutrophil extracellular traps from neutrophils not having released neutrophil extracellular traps, and therefore has difficulty in causing the normal neutrophils and the neutrophil extracellular traps to form their respective independent clusters on a scattergram.
The invention provides a new technique capable of detecting neutrophil extracellular traps with high accuracy.
The present invention is defined by the appended claims. Also disclosed are the following embodiments.
In the specimen analyzing method of the present invention, a reagent may comprise the nucleic acid staining fluorescent dye.
According to the invention, it is possible to provide a new technique capable of detecting neutrophil extracellular traps with high accuracy.

BRIEF DESCRIPTION OF DRAWINGS

Fig. 1 is a schematic diagram of a scattergram of a biological specimen containing neutrophil extracellular traps according to a specimen analyzing method.
Fig. 2 is a block diagram illustrating an overall configuration of a specimen analyzer.
Fig. 3 is a schematic diagram illustrating a configuration of a detector of the specimen analyzer.
Fig. 4 is a flowchart of a processing procedure for the specimen analyzer to detect neutrophil extracellular traps.
Fig. 5 is a flowchart of a processing procedure for the specimen analyzer.
Fig. 6 is a flowchart of the processing procedure for the specimen analyzer to prepare a measurement specimen.
Fig. 7 is a flowchart of a processing procedure for the specimen analyzer to measure fluorescence and scattered light.
Fig. 8 is a flowchart of a processing procedure for the specimen analyzer to detect neutrophil extracellular traps.
Fig. 9 is a view for explaining a configuration of a reagent kit.
Fig. 10 presents scattergrams of a measurement specimen and a measurement control specimen in Example 1.
Fig. 11 presents scattergrams of a measurement specimen and a measurement control specimen in Comparative Example 1.
Fig. 12 is a graph presenting a ratio of particles appearing in a target location in a scattergram of each of the measurement specimen in Example 1, the measurement control specimen in Example 1, the measurement specimen in Comparative Example 1 and the measurement control specimen in Comparative Example 1.
Fig. 13 presents scattergrams of a measurement specimen and a measurement control specimen in Example 10.

EMBODIMENTS

1. Explanation of Terms

In Description, a numerical value range defined by specific endpoint values, such as "X to Y", includes all the values and rational numbers within the range and the specified endpoint values.
In Description, "particles after releasing neutrophil extracellular traps" mean remaining particles of neutrophils

after the neutrophils release neutrophil extracellular traps.

2. Specimen Analyzing Method

According to one or more embodiments, the present invention provides a specimen analyzing method for detecting neutrophil extracellular traps, wherein the method comprises : a step (A) of preparing a measurement specimen from a biological specimen containing blood cells by staining, with a nucleic acid staining fluorescent dye, nucleic acids contained in neutrophils not having released neutrophil extracellular traps; a step (B) of obtaining optical information including fluorescence information by irradiating the measurement specimen with light; and a step (C) of detecting, as neutrophil extracellular traps on the basis of the optical information, remaining particles of neutrophils after the neutrophiles release neutrophil extracellular traps, wherein the remaining particles of neutrophils after the neutrophils realease neutrophil extracelullar traps have lower fluorescence intensity than fluorescence intensity obtained from the neutrophils not having released neutrophil extracellular traps. The specimen analyzing method according to the present embodiment may be carried out using, for example, a specimen analyzer to be described later, reagents to be described later, and so on.

The neutrophil extracellular traps have been known to play important roles in protection against infection from bacteria and the like, thrombus, cancer metastasis, autoimmune diseases, and others. For this reason, it is clinically significant to detect the presence or absence of neutrophil extracellular traps in a sample. For example, in immunosuppression of a patient after transplantation of cells of an organ, bone marrow, or the like, a treatment for suppressing excessive inflammation, such as an administration of a steroid, can be carried out timely by detecting the generation of neutrophil extracellular traps in the blood of the patient. In the specimen analyzing method according to the present embodiment, the execution of the following operation in the step (A) enables good formation of a cluster of particles after releasing neutrophil extracellular traps. Thus, the presence of neutrophil extracellular traps in a sample can be detected with high accuracy. In addition, the number of neutrophil extracellular traps in the sample can be also counted with high accuracy. Specifically, in the step (A), nucleic acids contained in neutrophils not having released neutrophil extracellular traps in a biological specimen containing blood cells are stained with a nucleic acid staining fluorescent dye. Thus, a measurement specimen can be obtained by the step (A). The particles after releasing neutrophil extracellular traps contain substantially no chromatin as a result of the release of the neutrophil extracellular traps. For this reason, the particles after releasing neutrophil extracellular traps are hardly stained with the nucleic acid staining fluorescent dye. Thus, in the measurement specimen, a difference between the fluorescence intensity of particles after releasing neutrophil extracellular traps and the fluorescence intensity of neutrophils not having released neutrophil extracellular traps is larger than in the case where the neutrophils not having released neutrophil extracellular traps are not stained. Thus, they can be distinguished from each other more clearly.

The biological specimen is a specimen containing blood cells. Examples of the biological specimen include, but are not limited to, blood, bone marrow aspirate, a lung lavage fluid, and the like. Among these biological specimens, the blood, the bone marrow aspirate, and the lung lavage fluid are preferred. As the blood cells, there are white blood cells, red blood cells, and platelets. In addition, as the white blood cells, there are neutrophils, eosinophils, basophil, lymphocytes, and monocytes.

In the step (A), it is possible to prepare a measurement specimen by damaging the cell membranes of neutrophils, and introducing a nucleic acid staining fluorescent dye. In the case where the cell membranes of neutrophils are damaged and the nucleic acid staining fluorescent dye is introduced in the step (A), the nucleic acids contained in the neutrophils can be more surely stained with the nucleic acid staining fluorescent dye.

The damaging to the cell membranes of neutrophils and the introducing of the nucleic acid staining fluorescent dye in the step (A) make it possible to make the fluorescence intensity obtained from neutrophils not having released neutrophil extracellular traps higher than the fluorescence intensity obtained from the remaining particles of neutrophils after the neutrophils realease neutrophil extracellular traps. Thus, the particles after releasing neutrophil extracellular traps can be more clearly distinguished from the neutrophils not having released neutrophil extracellular traps.

In the step (A), the damaging to the cell membranes of neutrophils and the introducing of the nucleic acid staining fluorescent dye may be carried out simultaneously, or in an order of the damaging to the cell membranes of neutrophils first, and the introducing of the nucleic acid staining fluorescent dye next.

The size of a damaged portion in the cell membrane of a neutrophil may be any size as long as the nucleic acid staining fluorescent dye can be introduced. The cell membranes of neutrophils may be damaged by using any of the following methods (A-1) to (A-3) and the like, for example. The method (A-1) is a method including mixing at least a biological specimen, a nucleic acid staining fluorescent dye, and an osmotic pressure regulator such that the osmotic pressure can be from 245 hPa to 1680 hPa. The method (A-2) is a method including mixing at least a biological specimen, a nucleic acid staining fluorescent dye, a surfactant, and an osmotic pressure regulator such that the osmotic pressure can be 2635 hPa or lower. The method (A-3) is a physical method such as electroporation or laser perforation.

In the method (A-1), at least a biological specimen, a nucleic acid staining fluorescent dye, and an osmotic

pressure regulator are mixed such that the osmotic pressure of the measurement specimen can be from 245 hPa to 1680 hPa. This makes damages on the cell membranes of neutrophils contained in the biological specimen, and causes the nucleic acid staining fluorescent dye to be introduced into the neutrophils from the damaged portions in the cell membranes.

Examples of the nucleic acid staining fluorescent dye include, but are not limited to, propidium iodide, ethidium bromide, ethidium-acridine heterodimer, ethidium diazide, ethidium homodimer-1, ethidium homodimer-2, ethidium monoazide, trimethylenebis[[3-[[4-[[(3-methylbenzothiazole-3-ium)-2-yl]methylene]-1,4-dihydroquin olin]-1-yl]propyl]dimethylaminium]•tetraiodide (TOTO-1), 4-[(3-methylbenzothiazole-2(3H)-ylidene)methyl]-1-[3-(trimethylaminio)propyl]quinolini um•diiodide (TO-PRO-1), N,N,N',N'-tetramethyl-N,N'-bis[3-[4-[3-[(3-methylbenzothiazol-3-ium)-2-yl]-2-propenylid ene]-1,4-dihydroquinolin-1-yl]propyl]-1,3-propanediaminium•tetraiodide (TOTO-3), 2-[3-[[1-[3-(trimethylaminio)propyl]-1,4-dihydroquinoline]-4-ylidene]-1-propenyl]-3-meth ylbenzothiazole-3-ium•diiodide (TO-PRO-3), fluorescent dyes represented by the following formulas (I) to (XI), and others.

[Fluorescent Dye Represented by Formula (I)]

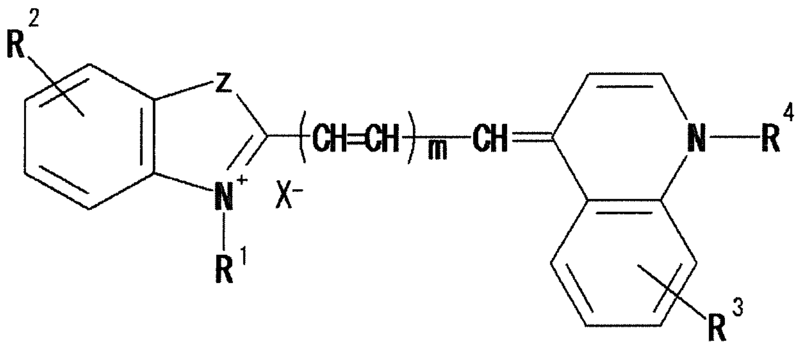

(I)

(In the formula, $R^1$ and $R^4$ each independently represent a hydrogen atom, an alkyl group which may contain a substituent, or a benzyl group which may contain a substituent; $R^2$ and $R^3$ each independently represent a hydrogen atom, a hydroxyl group, a halogen atom, an alkyl group, an alkenyl group, an alkynyl group, an alkoxy group, an alkylsulfonyl group, or a phenyl group; Z represents a sulfur atom, an oxygen atom, or an alkylene group which may contain a substituent; m represents a natural number of 0 to 3; and $X^-$ represents an anion.)

Note that, in the formula (I), if any one of $R^1$ and $R^4$ is an alkyl group having 6 to 18 carbon atoms, the other is preferably a hydrogen atom or an alkyl group having less than 6 carbon atoms. Among the alkyl groups having 6 to 18 carbon atoms, alkyl groups having 6, 8, or 10 carbon atoms are preferred. Examples of substituents on the alkyl group include, but are not limited to, a hydroxyl group, an ether group, an ester group, and the like. Examples of substituents on the benzyl group in $R^1$ and $R^4$ include, but are not limited to, an alkyl group having 1 to 20 carbon atoms ,an alkenyl group having 2 to 20 carbon atoms, an alkynyl group having 2 to 20 carbon atoms, and the like. Among the substituents in the benzyl group, a methyl group and an ethyl group are preferred. As an alkynyl group in $R^2$ and $R^3$, there are alkynyl groups having 2 to 20 carbon atoms. As an alkoxy group in $R^2$ and $R^3$, there are alkoxy groups having 1 to 20 carbon atoms. Among these alkoxy groups, a methoxy group and an ethoxy group are preferred. In Z, the number of carbon atoms in the alkylene group is one or two. As substituents on the alkylene group, there are a methylene group and an ethylene group. Examples of an anion in $X^-$ include, but are not limited to, halide ions such as fluoride ion, chloride ion, bromide ion, and iodide ion; trifluoromethanesulfonate ion ($CF_3SO_3^-$), borofluoride ion ($BF_4^-$), and the like.

[Fluorescent Dye Represented by Formula (II)]

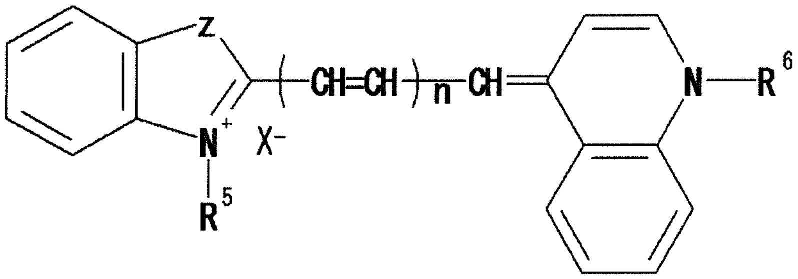

(II)

(In the formula, $R^5$ and $R^6$ each represent a lower alkyl group, $X^-$ represents an anion, Z represents a sulfur atom, an oxygen atom, or an alkylene group which may contain a substituent, and n represents 1 or 2.) In the formula (II), $R^5$ and $R^6$ may be the same as or different from each other. In the formula (II), the "lower alkyl group" is an alkyl group having 1 to 6 carbon atoms. Examples of an alkyl group having 1 to 6 carbon atoms include, but are not limited to, a methyl group, an ethyl group, a propyl group, a butyl group, an isobutyl group, a sec-butyl group, a tert-butyl group, a pentyl group, a hexyl group, and the like. Among these alkyl groups, the methyl group and the ethyl group are preferred. In the formula (II), the alkylene group which may contain a substituent is the same as the alkylene group which may contain a substituent in the formula (I). In addition, as Z, the sulfur atom is preferred. Examples of the anion in $X^-$ include, but are not limited to, halide ions; boron halide ions such as borofluoride ion ($BF_4^-$), boron chloride ion ($BCl_4^-$), and boron bromide ion ($BBr_4^-$); phosphorus compound ions; halogen oxoacid ions; fluorosulfate ions; methyl sulfate ions; tetraphenylboron compound ion having an aromatic ring halogen or a halogenoalkyl group as a substituent; and the like. Among these anions, the iodine ions are preferred. Among fluorescent dyes represented by the formula (II), the fluorescent dye NK-321 represented by the following formula (IIa) is preferred:

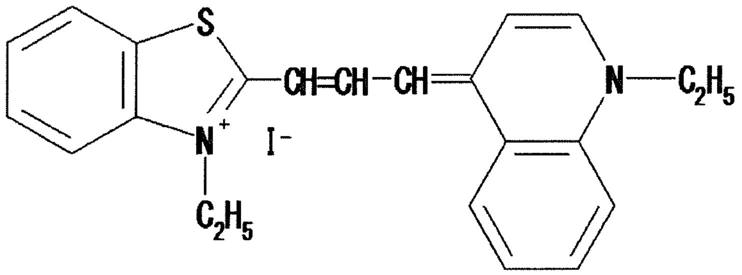

(IIa)

[Fluorescent Dye Represented by Formula (III)]

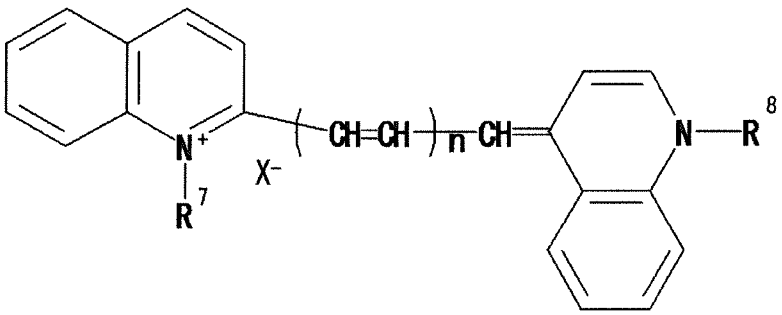

(III)

(In the formula, $R^7$ and $R^8$ each represent a lower alkyl group, $X^-$ represents an anion, and n represents 1 or 2.) In the formula (III), the lower alkyl group and the anion are the same as the lower alkyl group and the anion in the formula (II).

Among fluorescent dyes represented by the formula (III), a fluorescent dye represented by the following formula (IIIa) is preferred:

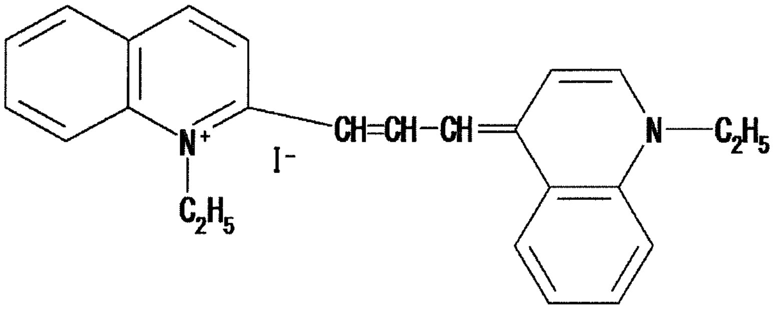

(IIIa)

[Fluorescent Dye Represented by Formula (IV)]

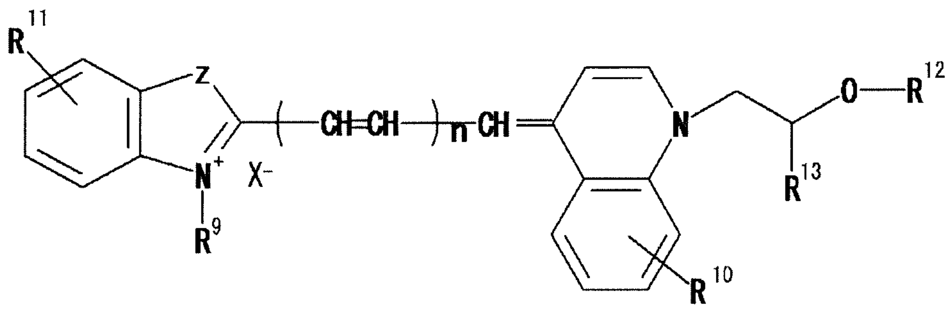

(IV)

(In the formula, $R^9$ represents a hydrogen atom or a lower alkyl group; $R^{10}$ and $R^{11}$ each independently represent a hydrogen atom, a lower alkyl group, or a lower alkoxy group; $R^{12}$ represents a hydrogen atom, an acyl group, or a lower alkyl group; $R^{13}$ represents a hydrogen atom, or a lower alkyl group which may contain a substituent; Z represents a sulfur atom, an oxygen atom, or an alkylene group which may contain a substituent; $X^-$ represents an anion, and n represents 1 or 2.)

In the formula (IV), the lower alkyl group, the anion, and the alkylene group which may contain a substituent are the same as the lower alkyl group, the anion, and the alkylene group which may contain a substituent in the formula (I). The lower alkoxy group is an alkoxy group having 1 to 6 carbon atoms. Examples of the alkoxy group having 1 to 6 carbon atoms include, but are not limited to, a methoxy group, an ethoxy group, a propoxy group, and the like. Among these alkoxy groups having 1 to 6 carbon atoms, the methoxy group and the ethoxy group are preferred. The acyl group is preferably an acyl group derived from an aliphatic carboxylic acid. Examples of the acyl group include, but are not limited to, an acetyl group, a propionyl group, and the like. Among these acyl groups, the acetyl group is preferred. In the lower alkyl group which may contain a substituent, examples of the substituents on the lower alkyl group include, but are not limited to, halogen atoms such as a fluorine atom, a chlorine atom, a bromine atom, and an iodine atom; hydroxyl groups, and the like. The lower alkyl group which may contain a substituent may contain 1 to 3 substituents. Among the lower alkyl groups which may contain a substituent, a lower alkyl group containing one hydroxyl group is preferred. It is preferable that Z be a sulfur atom, and $X^-$ be a bromide ion or a borofluoride ion ($BF_4^-$).

Among fluorescent dyes represented by the formula (IV), fluorescent dyes represented by the following formulas (IVa) to (IVc) are preferred:

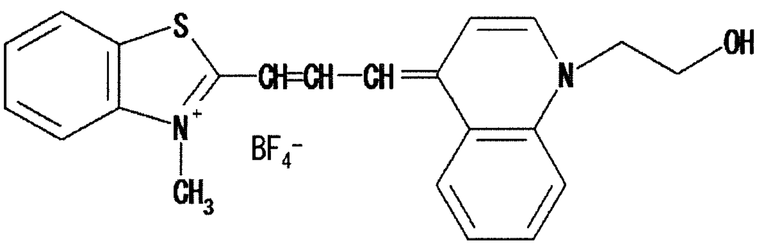

(IVa)

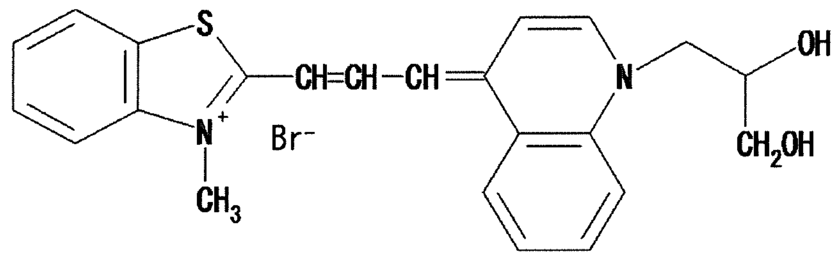

(IVb)

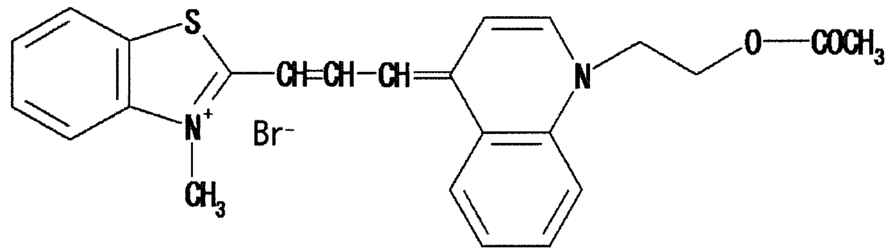

(IVc)

[Fluorescent Dye Represented by Formula (V)]

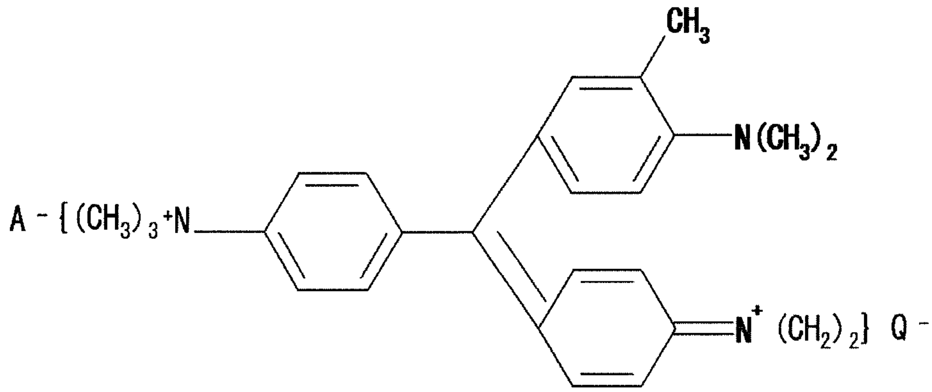

(V)

(In the formula, $A^-$ and $Q^-$ each independently represent a chloride ion ($Cl^-$) or an iodide ion ($I^-$).)

[Fluorescent Dye Represented by Formula (VI)]

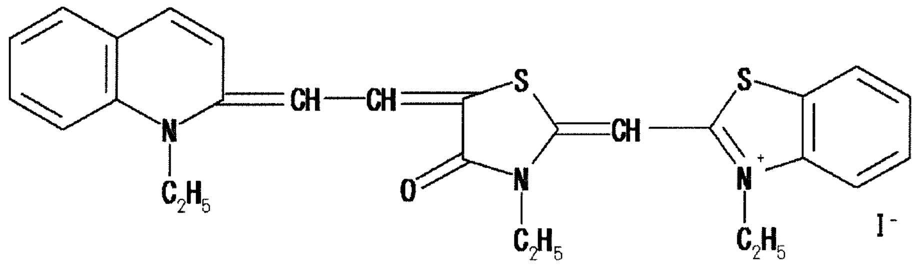

(VI)

[Fluorescent Dye NK-1570 Represented by Formula (VII)]

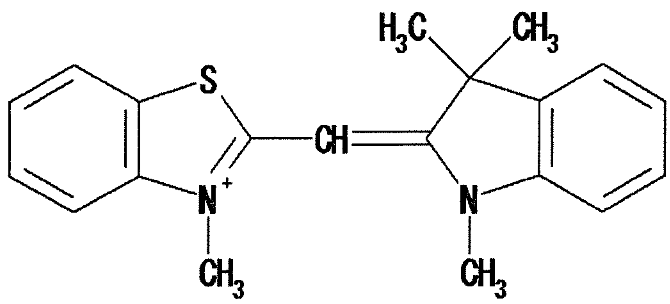

(VII)

[Fluorescent Dye NK-1049 Represented by Formula (VIII)]

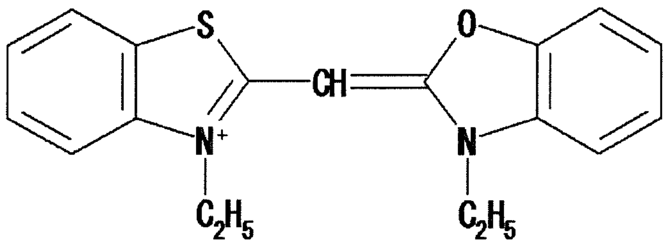

(VIII)

[Fluorescent Dye NK-98 Represented by Formula (IX)]

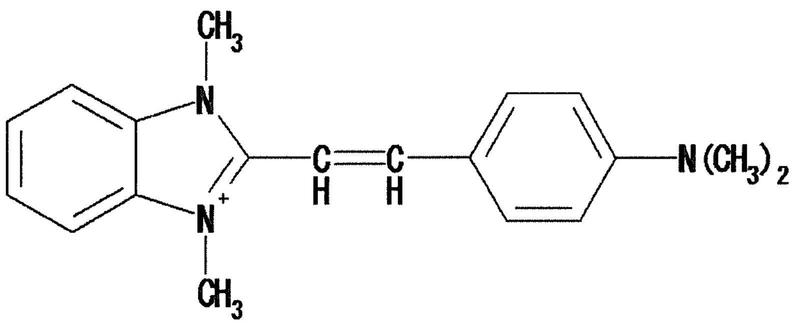

(IX)

[Fluorescent Dye NK-141 Represented by Formula (X)]

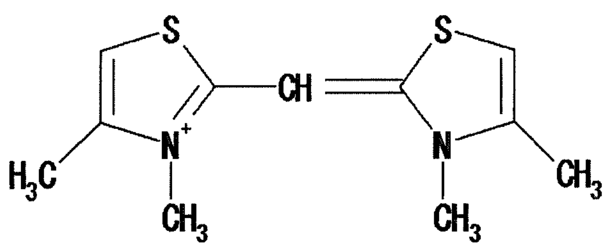

(X)

[Fluorescent Dye NK-321 Represented by Formula (XI)]

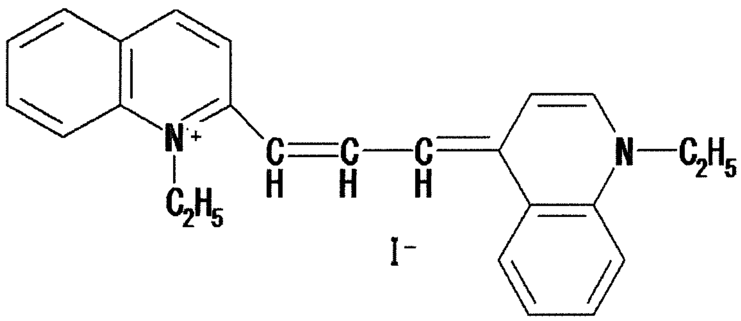

(XI)

Among these nucleic acid staining fluorescent dyes, the fluorescent dye NK-321 represented by the formula (XI) is preferred.

The nucleic acid staining fluorescent dye can be used by being dissolved in an appropriate solvent. The solvent may be, but is not limited to, any of water, organic solvents, and mixtures of them. Examples of the organic solvents include, but are not limited to, alcohol, ethylene glycol, diethylene glycol, triethylene glycol, dimethyl sulfoxide, and the like.

An amount of the nucleic acid staining fluorescent dye to be mixed with a biological specimen may be any amount sufficient to stain the nucleic acids contained in the blood cells. The amount of a nucleic acid staining fluorescent dye to be mixed with a biological specimen may be an amount excessive relative to the nucleic acids contained in the blood cells. In general, the amount of a nucleic acid staining fluorescent dye per $10^4$ white blood cells or $10^5$ red blood cells, which are blood cells suspended in 1 mL of isotonic solution, is preferably 0.5 μM or more, and more preferably 1 μM or more with the view of sufficiently staining the nucleic acids contained in the blood cells.

As used herein, osmotic pressure values refer to the osmotic pressure at a temperature of 25°C, unless indicated otherwise. The osmotic pressure is preferably determined using osmometry. For example, in one embodiment of the present invention the Automatic Osmometer 3250 (Advanced Instruments, Inc.; see also http://www.riko-trading.co.jp/advanced.html) can be used as a measuring device to determine the osmotic pressure.

As the osmotic pressure regulator, any reagent for regulating the osmotic pressure of a measurement specimen may be used, and examples thereof include, but are not limited to: a solution in which a sugar, an amino acid, sodium chloride, or the like is dissolved in a solvent; an organic solvent; and the like. Examples of the sugar include, but are not limited to, monosaccharides such as glucose and fructose; polysaccharides such as arabinose; sugar alcohols such as xylitol, sorbitol, mannitol, and ribitol; and the like. Examples of the amino acid include, but are not limited to, alanine, proline, glycine, valine, and the like. Examples of the solvent include, but are not limited to, water; buffer solutions such as a phosphate buffer solution, a citrate buffer solution, and a 4-(2-hydroxyethyl)-1-piperazineethanesulfonic acid (HEPES) buffer solution; and the like. Examples of the organic solvent include, but are not limited to, ethylene glycol, glycerin, and the like. Specific examples of the osmotic pressure regulator include, but are not limited to, a phosphate buffered saline, and the like.

An amount of an osmotic pressure regulator to be mixed with a biological specimen is such an amount that the osmotic pressure of the measurement specimen can be in a range of 249 hPa to 1680 hPa, both inclusive. The osmotic pressure of the measurement specimen is 249 hPa or higher and is preferably 272 hPa or higher with the view of improving the sensitivity in detection of neutrophil extracellular traps, and is 1680 hPa or lower and is preferably 568 hPa or lower with the same view. Due to an influence of such osmotic pressure, neutrophil extracellular traps are considered to be separated from the particles after releasing the neutrophil extracellular traps.

In the method (A-1), the measurement specimen may be further blended with an aromatic organic acid. Examples of the aromatic organic acid include, but are not limited to, a phthalic acid, a benzoic acid, a salicylic acid, a hippuric acid, a p-aminobenzenesulfonic acid, a benzenesulfonic acid, salts thereof, and the like. The aromatic organic acids may be each used alone, or may be used as a mixture of two or more kinds.

In the method (A-1), the pH of the measurement specimen is preferably 5.0 to 9.0 with the view of efficiently damaging the cell membranes of neutrophils. The pH of the measurement specimen can be adjusted by a pH adjuster such as sodium hydroxide or hydrochloric acid, for example. Here, if a buffer solution is used as a solvent for an osmotic pressure regulator, the pH of the measurement specimen can be adjusted by the osmotic pressure regulator. Moreover, if an aromatic organic acid is used in the method (A-1), the pH of the measurement specimen can be adjusted by the aromatic organic acid.

In the method (A-1), after the biological specimen, the nucleic acid staining fluorescent dye, the osmotic pressure regulator, and an optional aromatic organic acid are mixed together, the measurement specimen may be incubated at 25 to 41°C for 0.25 to 3 minutes with the view of more surely damaging the cell membranes of neutrophils and introducing the nucleic acid staining fluorescent dye.

In the method (A-1), an auxiliary agent other than the nucleic acid staining fluorescent dye and the osmotic

pressure regulator may be used as needed. Examples of the auxiliary agent include, but are not limited to, a pH adjuster and the like.

In the method (A-2), at least the biological specimen, the nucleic acid staining fluorescent dye, the surfactant, and the osmotic pressure regulator are mixed such that the osmotic pressure of the measurement specimen can be 2635 hPa or lower. This makes damages on the cell membranes of the blood cells such as neutrophils contained in the biological specimen, and causes the nucleic acid staining fluorescent dye to be introduced into the blood cells such as neutrophils from the damaged portions.

The nucleic acid staining fluorescent dyes and the osmotic pressure regulators usable in the method (A-2) are the same as the nucleic acid staining fluorescent dyes and the osmotic pressure regulators usable in the method (A-1). In the method (A-2), an amount of the nucleic acid staining fluorescent dye to be mixed with a biological specimen is equal to the amount of the nucleic acid staining fluorescent dye to be mixed with a biological specimen in the method (A-1).

An amount of the osmotic pressure regulator to be mixed with the biological specimen is such an amount that the osmotic pressure of the measurement specimen can be in a range of 2635 hPa or lower.

Examples of the surfactant include, but are not limited to, cation surfactants, nonion surfactants, and the like.

Examples of the cationic surfactant include, but are not limited to, a quaternary ammonium salt type surfactant, a pyridinium salt type surfactant, and the like. An example of the quaternary ammonium salt type surfactant is, but is not limited to, a surfactant represented by the following formula (XII):

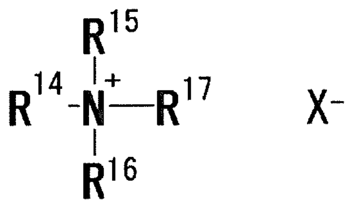

(XII)

,

(in the formula, $R^{14}$ represents an alkyl group having 6 to 18 carbon atoms or an alkenyl group having 6 to 18 carbon atoms; $R^{15}$ and $R^{16}$ each independently represent an alkyl group having 1 to 4 carbon atoms or an alkenyl group having 1 to 4 carbon atoms; $R^{17}$ represents an alkyl group having 1 to 4 carbon atoms, an alkenyl group having 1 to 4 carbon atoms, or a benzyl group; $X^-$ represents a halogen atom, and the total number of carbon atoms is 9 to 30), or the like.

In the formula (XII), for $R^{14}$, alkyl groups having 6, 8, 10, 12, or 14 carbon atoms and alkenyl groups having 6, 8, 10, 12, or 14 carbon atoms are preferred, and straight chain alkyl groups having 6, 8, 10, 12, or 14 carbon atoms are more preferred. Specific examples of $R^{14}$ include, but are not limited to, an octyl group, a decyl group, a dodecyl group, and the like. For $R^{15}$ and $R^{16}$, a methyl group, an ethyl group, and a propyl group are preferred. For $R^{17}$, a methyl group, an ethyl group, and a propyl group are preferred.

An example of the pyridinium salt type surfactant is, but is not limited to, a surfactant represented by the following formula (XIII):

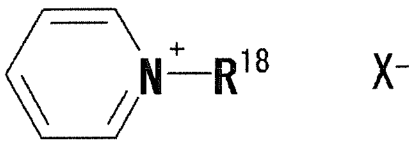

(XIII)

,

(in the formula, $R^{18}$ represents an alkyl group having 6 to 18 carbon atoms or an alkenyl group having 6 to 18 carbon atoms, and $X^-$ represents a halogen ion), or the like.

In the formula (XIII), for $R^{18}$, alkyl groups having 6, 8, 10, 12, or 14 carbon atoms and alkenyl groups having 6, 8, 10, 12, or 14 carbon atoms are preferred, and straight chain alkyl groups having 6, 8, 10, 12, or 14 carbon atoms are more preferred. Specific examples of $R^{18}$ include, but are not limited to, an octyl group, a decyl group, a dodecyl group, and the like.

An example of the nonionic surfactant is, but is not limited to, a polyoxyethylene-based nonionic surfactant represented by the following formula (XIV):

$$R^{19}-R^{20}\!-\!(CH_2CH_2O)_o\!-\!H \qquad (XIV)$$

,

(in the formula, $R^{19}$ represents an alkyl group having 8 to 25 carbon atoms, an alkenyl group having 8 to 25 carbon atoms, or an alkynyl group having 8 to 25 carbon atoms; $R^{20}$ represents an oxygen atom, an ester bond, or an oxyphenylene group; and o represents an integer of 10 to 50), or the like.

Specific examples of the nonionic surfactant include, but are not limited to, polyoxyethylene alkyl ether, polyoxyethylene sterol, polyoxyethylene castor oil, polyoxyethylene sorbitol fatty acid ester, polyoxyethylene alkylamine, polyoxyethylene polyoxypropylene alkyl ether, and the like.

These surfactants may be each used alone, or may be used as a mixture of two or more kinds. In the case of using a mixture of two or more kinds of surfactants, any combination may be selected from a combination of a cationic surfactant and a nonionic surfactant, a combination of cationic surfactants and a combination of nonionic surfactants.

The surfactant may be in the form of a solution. Examples of a solvent in which the surfactant is dissolved include, but are not limited to, water, an organic solvent, a mixture of water and an organic solvent, and the like. Examples of the organic solvent include, but are not limited to, alcohol, ethylene glycol, dimethyl sulfoxide, and the like.

An amount of a surfactant to be mixed with a biological specimen may be any amount as long as the amount of the surfactant contained in the measurement specimen can be a predetermined amount. In general, if the surfactant is a cationic surfactant, the amount of the surfactant in the measurement specimen is preferably 10 to 10000 ppm, and is more preferably 100 to 1000 ppm. In general, if the surfactant is a nonionic surfactant, the amount of the surfactant in the measurement specimen is preferably 10 to 100000 ppm, is more preferably 100 to 10000 ppm, and is even more preferably 1000 to 5000 ppm.

In the method (A-2), the measurement specimen may be further blended with an aromatic organic acid. Examples of the aromatic organic acid include, but are not limited to, a phthalic acid, a benzoic acid, a salicylic acid, a hippuric acid, a p-aminobenzenesulfonic acid, a benzenesulfonic acid, salts thereof, and the like. The aromatic organic acids may be each used alone, or may be used as a mixture of two or more kinds.

In the method (A-2), the pH of the measurement specimen is preferably 5.0 to 9.0 with the view of efficiently damaging the cell membranes of neutrophils. The pH of the measurement specimen can be adjusted by a pH adjuster such as sodium hydroxide or hydrochloric acid, for example. Here, if a buffer solution is used as a solvent for an osmotic pressure regulator, the pH of the measurement specimen can be adjusted by the osmotic pressure regulator. Moreover, if an aromatic organic acid is used in the method (A-2), the pH of the measurement specimen can be adjusted by the aromatic organic acid.

In the method (A-2), after the biological specimen, the nucleic acid staining fluorescent dye, the osmotic pressure regulator, and an optional aromatic organic acid are mixed together, the measurement specimen may be incubated at 25 to 41°C for 0.25 to 3 minutes with the view of more surely damaging the cell membranes of neutrophils and introducing the nucleic acid staining fluorescent dye.

In the method (A-2), an auxiliary agent other than the nucleic acid staining fluorescent dye, the surfactant, and the osmotic pressure regulator may be used as needed. The auxiliary agent usable is the same as the auxiliary agent usable in the method (A-1).

Next, in the step (B), the fluorescence information is obtained by irradiating the measurement specimen with light. The step (B) can be carried out by using, for example, a flow cytometer.

The light with which the measurement specimen is irradiated can be selected as appropriate depending on the kind of a nucleic acid staining fluorescent dye used.

The fluorescence information is information obtained from nucleic acids stained with the nucleic acid staining fluorescent dye. An example of the fluorescence information is, but is not limited to, fluorescence intensity or the like. The higher the fluorescence intensity, the more the nucleic acids stained with the nucleic acid staining fluorescent dye. The difference in fluorescence intensity can be used to sort white blood cells, to determine the presence or absence of the generation of neutrophil extracellular traps, or to do the like.

In the step (B), scattered light information can be further obtained by irradiating the measurement specimen with light. In other words, the optical information including the fluorescence information and the scattered light information can be obtained by irradiating the measurement specimen with light. As the scattered light information, there are forward scattered light information and side scattered light information. If particles such as blood corpuscles are present as obstacles in a traveling direction of light, the light changes in its traveling direction due to the particles and thereby generates scattered light. The detection of scattered light makes it possible to obtain information on the sizes and substance properties of the particles. From the forward scattered light, it is possible to obtain information on the sizes of particles such as blood cells. Meanwhile, from the side scattered light, it is possible to obtain internal information on particles. The intensity of the side scattered light generated by irradiating cells with laser light reflects the intracellular

complexity such, for example, as the shape of the nucleus, the size of the nucleus, the density, and the amount of granules. Thus, the intensity of the scattered light can be used to sort while blood cells or do the like. As the scattered light information, the side scattered light information is preferred from the viewpoint of more surely sorting out the particles after releasing neutrophil extracellular traps.

Thereafter, in the step (C), particles after releasing neutrophil extracelullar traps having lower fluorescence intensity than the fluorescence intensity obtained from neutrophils not having released neutrophil extracellular traps are detected as neutrophil extracellular traps on the basis of the fluorescence information. Specifically, for example, particles having lower fluorescence intensity than the average value of the fluorescence intensity of the cluster of the neutrophils not having released neutrophil extracellular traps can be detected as particles after releasing neutrophil extracellular traps. Instead, particles having lower fluorescence intensity than the mode of the fluorescence intensity in the cluster of the neutrophils not having released neutrophil extracellular traps can be detected as the particles after releasing neutrophil extracellular traps.

If the scattered light information is additionally obtained in the step (B), neutrophil extracellular traps can be detected on the basis of the fluorescence information and the scattered light information in the step (C). In this case, in the step (C), particles having lower fluorescence intensity than the fluorescence intensity obtained from neutrophils not having released neutrophil extracellular traps and having higher scattered light intensity than the scattered light intensity obtained from the lymphocytes can be detected as the particles after releasing neutrophil extracellular traps. More specifically, for example, particles having lower fluorescence intensity than the average value of the fluorescence intensity in the cluster of neutrophils not having released neutrophil extracellular traps, and having higher scattered light intensity than the average value of the scattered light intensity in the cluster of lymphocytes can be detected as particles after releasing neutrophil extracellular traps. Instead, particles having lower fluorescence intensity than the mode of the fluorescence intensity in the cluster of neutrophils not having released neutrophil extracellular traps, and having higher scattered light intensity than the mode of the scattered light intensity in the cluster of lymphocytes can be detected as particles after releasing neutrophil extracellular traps.

In the case of using a scattergram by flow cytometry, detection area A1, detection area A2, and detection area A3 appear on the scattergram as presented in Fig. 1. Detection area A1 is an area where a cluster of particles after releasing neutrophil extracellular traps appears. Detection area A2 is an area where a cluster of neutrophils not having released neutrophil extracellular traps appears. Detection area A3 is an area where a cluster of lymphocytes appears. Detection area A1 exhibits lower fluorescence intensity than the fluorescence intensity in detection area A2. Detection area A1 exhibits higher scattered light intensity than the scattered light intensity in detection area A3. If particles are detected in detection area A1, it can be determined that the neutrophil extracellular traps have been released from neutrophils. Note that it is considered that the neutrophil extracellular traps themselves are dissolved in a liquid component of the measurement specimen, or appear on the scattergram as debris components significantly smaller than the neutrophils.

[Overall Configuration of Specimen Analyzer]

With reference to the accompanying drawings, description is provided for an example of a specimen analyzer (hereinafter also simply referred to as "analyzer") for use in the foregoing specimen analyzing method. As illustrated in Fig. 2, analyzer 10 includes measurement unit 20 and information processing unit 30. Measurement unit 20 and information processing unit 30 are communicatively connected to each other.

[Configuration of Measurement Unit]

As illustrated in Fig. 2, measurement unit 20 includes specimen preparation part 100, detector 200, reagent storage 300, biological specimen storage 400 which stores biological specimens, and controller 500.

Specimen preparation part 100 obtains reagents respectively from first container 301 and second container 302 in reagent storage 300. In addition, specimen preparation part 100 obtains a biological specimen from biological specimen storage 400. Moreover, specimen preparation part 100 mixes together the reagents and the biological specimen thus obtained. In this way, specimen preparation part 100 stains, with a nucleic acid staining fluorescent dye, nucleic acids contained in neutrophils not having released neutrophil extracellular traps in the biological specimen containing blood cells, thereby preparing a measurement specimen. Specimen preparation part 100 includes chamber 90, biological specimen transporter 111, first reagent transporter 112, second reagent transporter 113, and measurement specimen transporter 131. Biological specimen transporter 111, first reagent transporter 112, second reagent transporter 113, and measurement specimen transporter 131 are connected to compressors, respectively.

Chamber 90 is connected to biological specimen storage 400 via biological specimen transporter 111. Biological specimen transporter 111 is a tube through which a biological specimen flows. Biological specimen transporter 111 takes a certain amount of the biological specimen from biological specimen storage 400 by way of pressure generated

by the compressor. Biological specimen transporter 111 discharges the certain amount of biological specimen thus taken into chamber 90 by way of the pressure generated by the compressor.

Chamber 90 is connected to first container 301 of reagent storage 300 via first reagent transporter 112. First reagent transporter 112 is a tube through which a first reagent flows. First reagent transporter 112 takes a certain amount of the first reagent from first container 301 of reagent storage 300 by way of pressure generated by the compressor. First reagent transporter 112 discharges the certain amount of the first reagent thus taken into chamber 90 by way of the pressure generated by the compressor.

Chamber 90 is connected to second container 302 of reagent storage 300 via second reagent transporter 113. Second reagent transporter 113 is a tube through which a second reagent flows. Second reagent transporter 113 takes a certain amount of the second reagent from second container 302 of reagent storage 300 by way of pressure generated by the compressor. Then, second reagent transporter 113 discharges the certain amount of the second reagent thus taken into chamber 90 by way of the pressure generated by the compressor.

Chamber 90 is connected to detector 200 via measurement specimen transporter 131. Measurement specimen transporter 131 is a tube through which a measurement specimen prepared in chamber 90 is transported to detector 200.

Detector 200 includes light emitter 201, light receiver 202, and sheath flow section 203. Light receiver 202 outputs, to calculator 31 of information processing unit 30, an electric signal depending on an amount of light received. In Fig. 3, detector 200 sends the measurement specimen and a sheath liquid to flow cell 231 in sheath flow section 203. In this way, detector 200 generates a liquid flow inside flow cell 231. In addition, detector 200 irradiates, with laser light, particles such as blood cells contained in the liquid flow passing inside flow cell 231. Moreover, detector 200 measures light obtained from the particles irradiated with the laser light.

Light emitter 201 includes light source 211 that emits laser light, collimator lens 212, condenser lens 213, and beam stopper 214. Light emitter 201 irradiates flow cell 231 with laser light emitted from light source 211 and traveling via collimator lens 212 and condenser lens 213. Light source 211 can be selected as appropriate depending on the kind of a nucleic acid staining fluorescent dye used and the type of laser light used. Examples of the laser light include, but are not limited to, semiconductor laser light such as red semiconductor laser light and blue semiconductor laser light; gas laser light such as argon laser light; and the like.

Light receiver 202 includes forward scattered light receiver 202a, side scattered light receiver 202b, and fluorescence receiver 202c. Instead, light receiver 202 may include fluorescence receiver 202c without including forward scattered light receiver 202a and side scattered light receiver 202b.

Forward scattered light receiver 202a includes forward condenser lens 221, pin hole 222, and photodiode 223. Forward scattered light receiver 202a condenses forward scattered light through forward condenser lens 221. Then, in forward scattered light receiver 202a, photodiode 223 receives light having passed through pin hole 222.

Side scattered light receiver 202b includes side condenser lens 224, dichroic mirror 225, and photodiode 226. Side scattered light receiver 202b condenses side scattered light through side condenser lens 224. Then, side scattered light receiver 202b causes dichroic mirror 225 to reflect part of the side scattered light. Moreover, in side scattered light receiver 202b, photodiode 226 receives light having been reflected by dichroic mirror 225.

Fluorescence receiver 202c includes spectral filter 227 and avalanche photodiode 228. In fluorescence receiver 202c, light having passed through dichroic mirror 225 passes through spectral filter 227. Then, in fluorescence receiver 202c, avalanche photodiode 228 receives the light having passed through spectral filter 227.

Sheath flow section 203 includes flow cell 231. Sheath flow section 203 is configured such that a measurement specimen enclosed in the sheath liquid flows in flow cell 231.

Returning to Fig. 2, reagent storage 300 includes first container 301 and second container 302. First container 301 contains the first reagent containing a nucleic acid staining fluorescent dye. Meanwhile, second container 302 contains the second reagent containing an osmotic pressure regulator and a surfactant. First container 301 and second container 302 are both provided with identifiers respectively identifying the kinds of reagents contained in the containers. Examples of the identifiers include, but are not limited to, barcodes and the like.

Biological specimen storage 400 includes biological specimen containers 401. Biological specimen containers 401 contain respectively different kinds of biological specimens. Moreover, biological specimen storage 400 transports biological specimen container 401 containing a desired biological specimen to a specimen aspiration position. Biological specimen containers 401 are all provided with identifiers respectively identifying the kinds of biological specimens contained in the containers. Examples of the identifiers include, but are not limited to, barcodes and the like.

Controller 500 includes CPU (Central Processing Unit) 501, and memory 502. Controller 500 is constituted by a computer.

Memory 502 stores computer programs, reagent identification information for identifying the reagents stored in reagent storage 300, specimen preparation information concerning preparation methods of measurement specimens, and specimen identification information for identifying the biological specimens stored in biological specimen storage 400. Examples of the computer programs include, but are not limited to, a computer program for preparing measurement specimens, a computer program for obtaining the fluorescence information and the scattered light information on measurement specimens, and the like. An example of the reagent identification information is, but is not limited to, information by which a kind of each reagent, the position of the container containing the reagent, and the identifier are associated with each other, or the like. Then, an example of the specimen identification information is, but is not limited to, information by which a kind of each biological specimen, the position of the container containing the biological specimen, and the identifier are associated with each other, or the like.

CPU 501 executes the computer program for preparing measurement specimens by using the reagent identification information and the specimen preparation information stored in memory 502. Thus, CPU 501 causes specimen preparation part 100 of measurement unit 20 to prepare a measurement specimen.

[Configuration of Information Processing Unit]

Information processing unit 30 includes calculator 31, display 32, and input section 33 as illustrated in Fig. 2. Information processing unit 30 detects, as neutrophil extracellular traps, particles after releasing neutrophil extracelullar traps having lower fluorescence intensity than the fluorescence intensity obtained from neutrophils not having released neutrophil extracellular traps, on the basis of the fluorescence information obtained by detector 200. In the present embodiment, information processing unit 30 is constituted by a computer system. Calculator 31 includes CPU 601 and memory 602. CPU 601 executes computer programs stored in memory 602. An example of display 32 is, but is not limited to, a screen display or the like. Display 32 displays, for example, information on the presence or absence of neutrophil extracellular traps and so on. Examples of input section 33 include, but are not limited to, a keyboard, a mouse, and the like.

In memory 602, computer programs such as an operating system and application programs to be executed by CPU 601 and data for use to execute the computer programs are installed. Examples of the application programs include, but are not limited to, a computer program for detecting neutrophil extracellular traps, and the like. CPU 601 executes the computer program, stored in memory 602, for detecting neutrophil extracellular traps. In this way, CPU 601 causes analyzer 10 to detect neutrophil extracellular traps.

The nucleic acid staining fluorescent dye, the osmotic pressure regulator, and the surfactant may be simultaneously mixed with the biological specimen. For this reason, the nucleic acid staining fluorescent dye, the osmotic pressure regulator, and the surfactant may be contained in a common container in place of first container 301 and second container 302. In this case, a single reagent transporter is used in place of first reagent transporter 112 and second reagent transporter 113.

[Processing Procedures of Specimen Analyzer]

On the basis of Fig. 4, outlines of processing procedures of analyzer 10 are described. In the following processing procedure, controller 500 of measurement unit 20 executes the computer program, stored in memory 502, for preparing measurement specimens by using the reagent identification information and the specimen preparation information fetched from memory 502. Moreover, controller 500 executes the computer program, stored in memory 502, for obtaining the fluorescence information and the scattered light information on measurement specimens. Calculator 31 of information processing unit 30 executes the computer program, stored in memory 602, for detecting neutrophil extracellular traps by using the optical data thus obtained.

In step S1, controller 500 of measurement unit 20 causes specimen preparation part 100 to prepare a measurement specimen. The preparation of the measurement specimen in step S1 is carried out in accordance with the later-described processing procedure presented in Figs. 5 and 6.

In step S2, controller 500 causes detector 200 to measure fluorescence and scattered light obtained from the measurement specimen. The measurement of the fluorescence and the scattered light in step S2 is carried out in accordance with the processing procedure presented in Fig. 7.

In step S3, calculator 31 of information processing unit 30 executes the computer program for detecting neutrophil extracellular traps, and thereby detects neutrophil extracellular traps.

[Processing Procedure for Measurement Specimen Preparation Step]

The outline of the measurement specimen preparation step by analyzer 10 is described with Figs. 5 and 6. The measurement specimen preparation step corresponds to the aforementioned step (A) of the specimen analyzing method.

As illustrated in Fig. 5, in step S101, controller 500 causes biological specimen storage 400 to transport desired biological specimen container 401 to the specimen aspiration position. In this step, controller 500 causes biological specimen storage 400 to select biological specimen container 401 containing a desired biological specimen on the basis of the specimen identification information stored in memory 502. Then, controller 500 causes biological specimen storage 400 to transport biological specimen container 401 thus selected so that biological specimen container 401 is located

at the specimen aspiration position.

In step S102, controller 500 causes specimen preparation part 100 to aspirate a certain amount of the biological specimen in biological specimen container 401. Specifically, controller 500 causes specimen preparation part 100 to aspirate the certain amount of the biological specimen from biological specimen container 401 via biological specimen transporter 111.

In step S103, controller 500 causes specimen preparation part 100 to discharge the biological specimen into chamber 90. Specifically, controller 500 causes specimen preparation part 100 to discharge the certain amount of the biological specimen aspirated into chamber 90 via biological specimen transporter 111.

As illustrated in Fig. 6, in step S104, controller 500 causes specimen preparation part 100 to aspirate a certain amount of the first reagent in first container 301. Specifically, controller 500 causes specimen preparation part 100 to aspirate the certain amount of the first reagent from first container 301 via first reagent transporter 112. Thereafter, in step S105, controller 500 causes specimen preparation part 100 to discharge the first reagent into chamber 90. Specifically, controller 500 causes specimen preparation part 100 to discharge the certain amount of the first reagent aspirated into chamber 90 via first reagent transporter 112.

In step S106, controller 500 causes specimen preparation part 100 to aspirate a certain amount of the second reagent in second container 302. After that, in step S107, controller 500 causes specimen preparation part 100 to discharge the second reagent to chamber 90. Steps S106 and S107 are the same as steps S104 and S105 in Fig. 6 except that a series of processes of aspirating and discharging the second reagent is performed via second reagent transporter 113. In the present embodiment, steps S106 and S107 are executed in parallel to steps S104 and S105 in Fig. 6.

In step S108, controller 500 causes specimen preparation part 100 to stir and mix the biological specimen, the first reagent, and the second reagent in chamber 90. In this way, nucleic acids contained in neutrophils not having released neutrophil extracellular traps in the biological specimen containing blood cells are stained with the nucleic acid staining fluorescent dye to obtain a measurement specimen.

After that, the processing advances to the measurement step of step S2 in Fig. 4.

[Processing Procedure for Measurement Step]

Next, the outline of the processing procedure for the fluorescence and scattered light measurement step by analyzer 10 is described with Fig. 7. The measurement step corresponds to the foregoing step (B) of the specimen analyzing method.

In step S201, controller 500 causes specimen preparation part 100 to introduce the measurement specimen into flow cell 231 of detector 200. Specifically, controller 500 causes specimen preparation part 100 to transport the measurement specimen in chamber 90 to flow cell 231 of detector 200 via measurement specimen transporter 131. Next, in step S202, controller 500 causes detector 200 to irradiate the measurement specimen with light. Specifically, controller 500 causes light emitter 201 of detector 200 to irradiate, with light, the measurement specimen flowing inside flow cell 231. Then, in step S203, controller 500 causes detector 200 to measure fluorescence and scattered light obtained from the measurement specimen. Specifically, controller 500 causes light receiver 202 of detector 200 to output optical data to calculator 31 of information processing unit 30, the optical data being of electric signals respectively depending on the intensity of fluorescence and the intensity of scattered light received by light receiver 202.

After that, the processing advances to the detection step of step S3 in Fig. 4.

[Processing Procedure for Detection Step]

The outline of the processing procedure for the neutrophil extracellular trap detection step by analyzer 10 is described with Fig. 8. The detection step corresponds to the foregoing step (C) of the specimen analyzing method.

In step S301, information processing unit 30 receives the optical data from measurement unit 20. Next, in step S302, CPU 601 sorts particles by using the received optical data. Specifically, CPU 601 plots the particles on a scattergram with fluorescence intensity and scattered light intensity by using the received optical data. By use of the fluorescence intensity and the scattered light intensity, the particles are roughly sorted into a particle group appearing in detection area A1, a particle group appearing in detection area A2, and a particle group appearing in detection area A3, as presented in the scattergram in Fig. 1. Next, in step S303 of Fig. 8, CPU 601 determines whether neutrophil extracellular traps are present. Specifically, CPU 601 extracts particles appearing in detection area A1. CPU 601 determines that neutrophil extracellular traps have been released from neutrophils if the particles are extracted in detection area A1 on the scattergram in Fig. 1. In this step, CPU 601 may determine whether neutrophil extracellular traps have been released from neutrophils, according to whether the number of particles appearing in detection area A1 is equal to or larger than a predetermined value. This predetermined value is a value that makes it possible to determine the absence of neutrophil extracellular traps by taking noise and the like into account. After that, CPU 601 outputs the detection result to display

32 and the like.

[Modification Examples of Processing Procedure]

The addition of the first reagent and the second reagent to the biological specimen may be carried out in the order of the addition of the first reagent and the addition of the second reagent. Specifically, a series of steps S106 and S107 may be carried out after a series of steps S104 and S105. Instead, the addition of the first reagent and the second reagent to the biological specimen may be carried out in the order of the addition of the second reagent and the addition of the first reagent. Specifically, a series of steps S104 and S105 may be carried out after a series of steps S106 and S107. A reagent containing a nucleic acid staining fluorescent dye, an osmotic pressure regulator, and a surfactant may be used instead of the first reagent and the second reagent.
In the case where light receiver 202 includes fluorescence receiver 202c without including forward scattered light receiver 202a and side scattered light receiver 202b, CPU 601 uses the received fluorescence intensity to detect, as neutrophil extracellular traps, particles after realeasing neutrophil extracelullar traps having lower fluorescence intensity than the fluorescence intensity obtained from neutrophils not having released neutrophil extracellular traps.

4. Reagent

Also disclosed is a reagent for use in the forgoing specimen analyzing method. The reagent according to the present disclosure contains an osmotic pressure regulator and a nucleic acid staining fluorescent dye.
The osmotic pressure regulator and the nucleic acid staining fluorescent dye usable in the reagent according to the present disclosure are the same as the osmotic pressure regulator and the nucleic acid staining fluorescent dye usable in the foregoing specimen analyzing method. The osmotic pressure regulator and the nucleic acid staining fluorescent dye may be contained in respectively different reagents, or may be contained in a single reagent.
The reagent according to the present disclosure may further contain a surfactant. The surfactant usable in the reagent according to the present disclosure is the same as the surfactant usable in the foregoing specimen analyzing method. In the case where the reagent according to the present disclosure contains a surfactant, the surfactant, the osmotic pressure regulator, and the nucleic acid staining fluorescent may be contained in respectively different reagents, or may be contained in a single reagent. In the case where the reagent according to the present disclosure is composed of a reagent containing an osmotic pressure regulator and a reagent containing a nucleic acid staining fluorescent dye, a surfactant may be contained in any one or both of the reagent containing the osmotic pressure regulator and the reagent containing the nucleic acid staining fluorescent dye.
The reagent according to the present disclosure may further contain an aromatic organic acid, another solvent, an auxiliary agent, and the like. The aromatic organic acid usable in the reagent according to the present disclosure is the same as the aromatic organic acid usable in the foregoing specimen analyzing method. Examples of the solvent include, but are not limited to, water; a buffer solution; an organic solvent; a mixture of at least two of them; and the like. Examples of the buffer solution include, but are not limited to, a phosphate buffer solution, a citrate buffer solution, a HEPES buffer solution, and the like. Examples of the organic solvent include, but are not limited to, alcohol, ethylene glycol, dimethyl sulfoxide, and the like. Examples of the auxiliary agent include, but are not limited to, a chelating agent, a preservative, and the like.
The reagent according to the present disclosure can be fabricated by blending the osmotic pressure regulator, the nucleic acid staining fluorescent dye, and the surfactant into an appropriate solvent(s) depending on the form(s) of the reagent(s).
The reagent according to the present disclosure can be provided as a reagent kit in which the reagent is sealed in a container. Fig. 9 illustrates an example of a reagent kit according to the present disclosure. Reagent kit 800 illustrated in Fig. 9 includes container 802 containing first reagent 801, container 804 containing second reagent 803, package insert 805, and box 806. First reagent 801 contains a nucleic acid staining fluorescent dye. Second reagent 803 contains an osmotic pressure regulator and a surfactant. Package insert 805 contains description of an operation procedure for detecting neutrophil extracellular traps using reagent kit 800. Box 806 houses container 802 containing first reagent 801, container 804 containing second reagent 803, and package insert 805 therein. Note that, the reagent kit illustrated in Fig. 9 is a kit in which first reagent 801 and second reagent 803 are sealed in respectively different containers. Instead, a mixture of first reagent 801 and second reagent 803 may be sealed in a single container.

[Examples]

Description of Abbreviations

PMA: Phorbol-12-Myristate-13-Acetate
NETs: Neutrophil Extracellular Traps

(Example 1 and Comparative Example 1)

PMA is mixed with 1000 μL of healthy volunteer peripheral blood to have a final concentration of 162 nM. The resulting mixture is allowed to stand at room temperature (25°C) for 3 hours, causing NETs. 17 μL of the resulting mixture, 1 μL of a nucleic acid staining fluorescent dye (manufactured by Thermo Fisher Scientific Co., Ltd., trade name: Sytox green stain), which is unable to permeate the cell membranes, and phosphate buffered saline (composition: 10 mM of phosphate buffer solution (pH 7.4) and 150 mM of sodium chloride) are mixed so that the osmotic pressure is 317.8 hPa. As a result, the cell membranes of blood cells such as neutrophils contained in the peripheral blood are damaged, and the nucleic acid staining fluorescent dye is introduced into the blood cells such as the neutrophils from the damaged portions. The obtained specimen is used as a measurement specimen in Example 1. In addition, an operation similar to that of the preparation for the measurement specimen in Example 1 is performed to obtain a measurement control specimen in Example 1, but purified water is used instead of PMA.

PMA is mixed with 1000 μL of healthy volunteer peripheral blood to have a final concentration of 162 nM. The resulting mixture is allowed to stand at room temperature (25°C) for 3 hours. 17 μL of the resulting mixture, 1 μL of the nucleic acid staining fluorescent dye, and the phosphate buffered saline are mixed so that the osmotic pressure is 6673.8 hPa, and a measurement specimen in Comparative Example 1 is thus obtained. In addition, an operation similar to that of the preparation for the measurement specimen in Comparative Example 1 is performed to obtain a measurement control specimen in Comparative Example 1, but purified water is used instead of PMA. Note that, since the osmotic pressure is 6673.8 hPa, the cell membranes of neutrophils contained in the measurement specimen and the measurement control specimen in Comparative Example 1 are substantially not damaged.

Using a flow cytometer, scattergrams are obtained by measuring fluorescence and scattered light from nucleic acids contained in each of the measurement specimens and the measurement control specimens.

Fig. 10 presents scattergrams of the measurement specimen and the measurement control specimen in Example 1. Fig. 11 presents scattergrams of the measurement specimen and the measurement control specimen in Comparative Example 1. In the drawings, (A) presents a relationship between the fluorescence intensity and the side scattered light intensity of the measurement control specimen, (B) presents a relationship between the fluorescence intensity and the side scattered light intensity of the measurement specimen, (C) presents a relationship between the fluorescence intensity and the forward scattered light intensity of the measurement control specimen, and (D) presents a relationship between the fluorescence intensity and the forward scattered light intensity of the measurement specimen.

From the results presented in Fig. 10, it is found that a cluster is observed in a right lower part of the scattergram of the measurement specimen in Example 1, whereas no cluster is observed in the scattergram of the measurement control specimen in Example 1. Thus, NETs are caused in the measurement specimen in Example 1. This suggests that cluster A at the right lower part in the scattergram of the measurement specimen in Example 1 in Fig. 10 is a cluster of particles after releasing NETs. In general, most of white blood cells in the healthy volunteer peripheral blood are lymphocytes and neutrophils. The lymphocytes are known to have lower side scattered light intensity than that of the neutrophils. In Fig. 10, cluster B at a left upper part in the scattergram of the measurement specimen in Example 1 is formed by a population of particles having lower side scattered light intensity, which suggests that cluster B is a cluster of lymphocytes. Moreover, cluster C at a right upper part in the scattergram of the measurement specimen in Example 1 is formed by a population of particles having higher side scattered light intensity, which suggests that cluster C is a cluster of neutrophils not having released NETs. On the basis of these results, it is found that a cluster of particles having lower fluorescence intensity than that of the neutrophils not having released NETs, and having higher scattered light intensity than that of the lymphocytes is a cluster of particles after releasing NETs.

On the other hand, from the results presented in Fig. 11, it is found that there is almost no difference between the scattergrams of the measurement specimen and the scattergrams of the measurement control specimen in Comparative Example 1. This suggests that, in both the measurement specimen and the measurement control specimen in Comparative Example, the blood cells such as neutrophils are not damaged, and the nucleic acid staining fluorescent dye is not introduced into the blood cells such as the neutrophils.

In each of the scattergrams of Figs. 10 and 11, a ratio of particles appearing in a target location is calculated in accordance with the following formula (XV):

$$\text{(Ratio of particles appearing in target location)} = \text{([Number of particles appearing in target location]/[Total number of particles in scattergram])} \qquad \text{(XV)}.$$

Note that the target location used is a location in which the fluorescence intensity is lower than the fluorescence intensity of neutrophils not having released NETs and the scattered light intensity is higher than the scattered light intensity of lymphocytes.

Fig. 12 presents the ratio of particles appearing in the target location in the scattergram of each of the measurement specimen in Example 1, the measurement control specimen in Example 1, the measurement specimen in Comparative Example 1, and the measurement control specimen in Comparative Example 1. In Fig. 12, lane 1 presents the ratio of particles appearing in the target location in the scattergram of the measurement specimen in Example 1, lane 2 presents the ratio of particles appearing in the target location in the scattergram of the measurement control specimen in Example 1, lane 3 presents the ratio of particles appearing in the target location in the scattergram of the measurement specimen in Comparative Example 1, and lane 4 presents the ratio of particles appearing in the target location in the scattergram of the measurement control specimen in Comparative Example 1.

From the results presented in Fig. 12, it is found that the ratio of particles appearing in the target location in Example 1, in which the nucleic acid staining fluorescent dye is introduced with the cell membranes of neutrophils damaged, is higher than that in Comparative Example 1. These results suggest that the NETs can be detected with high accuracy by introducing the nucleic acid staining fluorescent dye with the cell membranes of neutrophils damaged.

(Examples 2 to 9 and Comparative Examples 2 to 5)

Measurement specimens in Examples 2 to 9 and Comparative Example 2 to 5 are obtained by performing an operation similar to that in Example 1, but 17 $\mu$L of the mixture containing healthy volunteer peripheral blood and PMA, the nucleic acid staining fluorescent dye (manufactured by Thermo Fisher Scientific Co., Ltd., trade name: Sytox green stain), and the phosphate buffered saline are mixed so that the osmotic pressure is 249.7 (Example 2), 272.4 (Example 3), 317.8 (Example 4), 385.9 (Example 5), 567.5 (Example 6), 885.3 (Example 7), 1203.1 (Example 8), 1679.8 (Example 9), 1974.9 (Comparative Example 2), 2860.2 (Comparative Example 3), 3473.1 (Comparative Example 4), or 6673.8 (Comparative Example 5). In addition, measurement control specimens of Examples 2 to 9 and of Comparative Example 2 to 5 are obtained by performing an operation similar to that of the preparation for the measurement specimens of Examples 2 to 9 and of Comparative Examples 2 to 5, but purified water is used in place of PMA.

Using the flow cytometer, scattergrams are obtained by measuring fluorescence and scattered light from nucleic acids contained in each of the measurement specimens and the measurement control specimens. A sensitivity for detecting NETs is obtained in accordance with the following formula (XVI):

$$\text{(Sensitivity for detecting NETs)} = \text{([Number A of particles appearing in target location]/[Number B of particles appearing in target location])} \qquad \text{(XVI)},$$

(in the formula, [Number A of particles appearing in target location] is the number of particles appearing in the target location in the scattergram of the measurement specimen, and [Number B of particles appearing in target location] is the number of particles appearing in the target location in the scattergram of the measurement control specimen). The number A of particles appearing in the target location and the number B of particles appearing in the target location are obtained in accordance with the formula (XV).

Table 1 presents a relationship between the osmotic pressure and the sensitivity for detecting NETs using the measurement specimens and the measurement control specimens in each of Examples 2 to 9 and Comparative Example 2 to 5.

[Table 1]

| Osmotic Pressure (hPa) | Sensitivity |
|---|---|
| 249.7 | 2.92 |
| 272.4 | 3.28 |
| 317.8 | 12.59 |
| 385.9 | 10.42 |
| 567.5 | 5.1 |
| 885.3 | 1.82 |
| 1203.1 | 3.82 |

(continued)

| Osmotic Pressure (hPa) | Sensitivity |
|---|---|
| 1679.8 | 1.86 |
| 1974.9 | 0.23 |
| 2860.2 | 1.27 |
| 3473.1 | 1.04 |
| 6673.8 | 1.23 |

From the results presented in Table 1, it is found that the sensitivity exceeds 1.8 in the case where the osmotic pressure of the measurement specimen is 245 to 1680 hPa. These results suggest that NETs can be detected with high accuracy in the case where the osmotic pressure of the measurement specimen is 245 to 1680 hPa.

(Example 10)

As the first reagent, a staining dye (trade name: STROMATOLYSER-4DS manufactured by Sysmex Corporation) is used. In addition, a mixture is obtained by adding dodecyltrimethylammonium chloride, polyoxyethylene (30) cetyl ether, potassium hydrogen phthalate, and ethylenediaminetetraacetic acid dipotassium salt to purified water to have the composition presented in Table 2. The pH of the resulting mixture is adjusted to pH 6.0 by using sodium hydroxide, thereby obtaining a second reagent. Then, 20 μL of peripheral blood, 20 μL of the first reagent, and 1000 μL of the second reagent are mixed. The resulting mixture is incubated at 40°C for 20 seconds to obtain a measurement specimen (osmotic pressure: 2633. 2 hPa).

[Table 2]

| Composition | |
|---|---|
| Dodecyltrimethylammonium Chloride (ppm) | 685 |
| Polyoxyethylene (30) Cetyl Ether (ppm) | 1750 |
| Potassium Hydrogen Phthalate (mM) | 40 |
| Ethylenediaminetetraacetic Acid Dipotassium Salt (g/L) | 0.2 |
| Sodium Hydroxide (g/L) | 1.431 |
| pH | 6 |

Using the flow cytometer, scattergrams are obtained by measuring fluorescence and scattered light from nucleic acids contained in each of the measurement specimen and the measurement control specimen.

Fig. 13 presents the scattergrams of the measurement specimen and the measurement control specimen of Example 10. In Fig. 13, (A) presents a relationship between the fluorescence intensity and the side scattered light intensity of the measurement control specimen, (B) presents a relationship between the fluorescence intensity and the side scattered light intensity of the measurement specimen, (C) presents a relationship between the fluorescence intensity and the forward scattered light intensity of the measurement control specimen, and (D) presents a relationship between the fluorescence intensity and the forward scattered light intensity of the measurement specimen.

From the results presented in Fig. 13, it is found that a cluster of particles after releasing NETs can be distinguished from the other clusters. Thus, it is found that NETs can be detected with higher accuracy by mixing a biological specimen, a nucleic acid staining fluorescent dye, and a surfactant so that the osmotic pressure is 2633.2 hPa or lower.

## Claims

**1.** A specimen analyzing method for detecting neutrophil extracellular traps, wherein the method comprises:

preparing a measurement specimen from a biological specimen containing blood cells by staining, with a nucleic acid staining fluorescent dye, nucleic acids contained in neutrophils not having released neutrophil extracellular traps;

obtaining optical information including fluorescence information by irradiating the measurement specimen with light; and

detecting, as neutrophil extracellular traps on the basis of the optical information, remaining particles of neutrophils after the neutrophils release neutrophil extracellular traps, wherein the remaining particles of neutrophils after the neutrophils release neutrophil extracellular traps have lower fluorescence intensity than fluorescence intensity obtained from the neutrophils not having released neutrophil extracellular traps.

**2.** The specimen analyzing method according to claim 1, wherein preparing the measurement specimen comprises damaging cell membranes of the neutrophils so as to introduce the nucleic acid staining fluorescent dye into the neutrophils.

**3.** The specimen analyzing method according to claim 1 or 2, wherein preparing the measurement specimen comprises, by damaging cell membranes of the neutrophils so as to introduce the nucleic acid staining fluorescent dye into the neutrophils, making the fluorescence intensity obtained from the neutrophils not having released neutrophil extracellular traps higher than fluorescence intensity obtained from the remaining particles of neutrophils after the neutrophils release neutrophil extracellular traps.

**4.** The specimen analyzing method according to any one of claims 1 to 3, wherein the optical information further includes scattered light information.

**5.** The specimen analyzing method according to claim 4, wherein the scattered light information comprises side scattered light information.

**6.** The specimen analyzing method according to claim 4 or 5, wherein detecting particles comprises detecting, as neutrophil extracellular traps, remaining particles of neutrophils after the neutrophils release neutrophil extracellular traps, wherein the remaining particles of neutrophils after the neutrophils release neutrophil extracellular traps have lower fluorescence intensity than the fluorescence intensity obtained from the neutrophils not having released neutrophil extracellular traps and have higher scattered light intensity than scattered light intensity obtained from lymphocytes.

**7.** The specimen analyzing method according to any one of claims 1 to 6, wherein obtaining the optical information comprises obtaining the optical information by a flow cytometer.

**8.** The specimen analyzing method according to any one of claims 1 to 7, wherein preparing the measurement specimen comprises mixing at least the biological specimen and the nucleic acid staining fluorescent dye so that an osmotic pressure is from 245 hPa to 1680 hPa.

**9.** The specimen analyzing method according to any one of claims 1 to 7, wherein preparing the measurement specimen comprises mixing at least the biological specimen, the nucleic acid staining fluorescent dye, and a surfactant so that an osmotic pressure is 2635 hPa or lower.

**10.** A specimen analyzer (10) for detecting neutrophil extracellular traps, wherein the specimen analyzer (10) comprises:

a specimen preparation part (100) that prepares a measurement specimen from a biological specimen containing blood cells by staining, with a nucleic acid staining fluorescent dye, nucleic acids contained in neutrophils not having released neutrophil extracellular traps;

a detector (200) that obtains fluorescence information by irradiating the measurement specimen with light; and

an information processing unit (30) that detects, as neutrophil extracellular traps, remaining particles of neutrophils after the neutrophils release neutrophil extracellular traps, wherein the remaining particles of neutrophils after the neutrophils release neutrophil extracellular traps have lower fluorescence intensity than fluorescence intensity obtained from the neutrophils not having released neutrophil extracellular traps.

**11.** The specimen analyzer (10) according to claim 10, wherein the detector (200) further obtains scattered light information by irradiating the measurement specimen with light.

**12.** The specimen analyzer (10) according to claim 11, wherein the scattered light information comprises side scattered light information.

13. The specimen analyzer (10) according to claim 11 or 12, wherein the information processing unit (30) detects, as the neutrophil extracellular traps, remaining particles of neutrophils after the neutrophils release neutrophil extracellular traps, wherein the remaining particles of neutrophils after the neutrophils release neutrophil extracellular traps have lower fluorescence intensity than the fluorescence intensity obtained from the neutrophils not having released neutrophil extracellular traps and have higher scattered light intensity than scattered light intensity obtained from lymphocytes.

14. The specimen analyzer (10) according to any one of claims 10 to 13, wherein the specimen preparation part (100) prepares the measurement specimen by mixing at least the biological specimen and the nucleic acid staining fluorescent dye so that an osmotic pressure is 245 to 1680 hPa.

15. The specimen analyzer (10) according to any one of claims 10 to 13, wherein the specimen preparation part (100) prepares the measurement specimen by mixing the biological specimen, the nucleic acid staining fluorescent dye, and a surfactant so that an osmotic pressure is 2635 hPa or lower.

**Patentansprüche**

1. Probenanalyseverfahren zum Nachweisen neutrophiler extrazellulärer Fallen (neutrophil extracellular traps), wobei das Verfahren umfasst:

   das Herstellen einer Messprobe aus einer biologischen Probe, die Blutzellen enthält, durch das Färben von Nukleinsäuren, die in Neutrophilen enthalten sind, die neutrophile extrazelluläre Fallen nicht freigesetzt haben, mit einem Nukleinsäure färbenden Fluoreszenzfarbstoff;
   das Erhalten von optischen Informationen einschließlich Fluoreszenzinformationen durch Bestrahlen der Messprobe mit Licht; und
   das Nachweisen, auf Basis der optischen Informationen, von verbleibenden Partikeln von Neutrophilen, nachdem die Neutrophile neutrophile extrazelluläre Fallen freisetzen, als neutrophile extrazelluläre Fallen, wobei die verbleibenden Partikel von Neutrophilen, nachdem die Neutrophile neutrophile extrazelluläre Fallen freisetzen, eine niedrigere Fluoreszenzintensität haben als die Fluoreszenzintensität, die von den Neutrophilen erhalten wird, die neutrophile extrazelluläre Fallen nicht freigesetzt haben.

2. Probenanalyseverfahren gemäß Anspruch 1, wobei das Herstellen der Messprobe das Beschädigen von Zellmembranen der Neutrophile umfasst, um den Nukleinsäure färbenden Fluoreszenzfarbstoff in die Neutrophile einzubringen.

3. Probenanalyseverfahren gemäß Anspruch 1 oder 2, wobei das Herstellen der Messprobe umfasst, dass die Fluoreszenzintensität, die von den Neutrophilen erhalten wird, die neutrophile extrazelluläre Fallen nicht freigesetzt haben, höher gemacht wird als die Fluoreszenzintensität, die von den verbleibenden Partikeln von Neutrophilen erhalten wird, nachdem die Neutrophile neutrophile extrazelluläre Fallen freisetzen, durch das Beschädigen von Zellmembranen der Neutrophile, um den Nukleinsäure färbenden Fluoreszenzfarbstoff in die Neutrophile einzubringen.

4. Probenanalyseverfahren gemäß irgendeinem der Ansprüche 1 bis 3, wobei die optischen Informationen weiter Streulichtinformationen einschließen.

5. Probenanalyseverfahren gemäß Anspruch 4, wobei die Streulichtinformationen Seitenstreulichtinformationen umfassen.

6. Probenanalyseverfahren gemäß Anspruch 4 oder 5, wobei das Nachweisen von Partikeln das Nachweisen von verbleibenden Partikeln von Neutrophilen, nachdem die Neutrophile neutrophile extrazelluläre Fallen freisetzen, als neutrophile extrazelluläre Fallen umfasst, wobei die verbleibenden Partikel von Neutrophilen, nachdem die Neutrophile neutrophile extrazelluläre Fallen freisetzen, eine niedrigere Fluoreszenzintensität haben als die Fluoreszenzintensität, die von den Neutrophilen erhalten wird, die neutrophile extrazelluläre Fallen nicht freigesetzt haben, und eine höhere Streulichtintensität als von Lymphozyten erhaltene Streulichtintensität haben.

7. Probenanalyseverfahren gemäß irgendeinem der Ansprüche 1 bis 6, wobei das Erhalten der optischen Informationen das Erhalten der optischen Informationen durch ein Durchflusszytometer umfasst.

8. Probenanalyseverfahren gemäß irgendeinem der Ansprüche 1 bis 7, wobei das Herstellen der Messprobe das Mischen mindestens der biologischen Probe und des Nukleinsäure färbenden Fluoreszenzfarbstoffs umfasst, so dass ein osmotischer Druck von 245 hPa bis 1680 hPa ist.

9. Probenanalyseverfahren gemäß irgendeinem der Ansprüche 1 bis 7, wobei das Herstellen der Messprobe das Mischen mindestens der biologischen Probe, des Nukleinsäure färbenden Fluoreszenzfarbstoffs und eines Tensids umfasst, so dass ein osmotischer Druck 2635 hPa oder niedriger ist.

10. Probenanalysator (10) zum Nachweisen neutrophiler extrazellulärer Fallen (neutrophil extracellular traps), wobei der Probenanalysator (10) umfasst:

ein Probenherstellungsteil (100), das eine Messprobe aus einer biologischen Probe herstellt, die Blutzellen enthält, durch das Färben von Nukleinsäuren, die in Neutrophilen enthalten sind, die neutrophile extrazelluläre Fallen nicht freigesetzt haben, mit einem Nukleinsäure färbenden Fluoreszenzfarbstoff;
einen Detektor (200), der Fluoreszenzinformationen durch Bestrahlen der Messprobe mit Licht erhält; und
eine Informationsverarbeitungseinheit (30), die verbleibende Partikel von Neutrophilen, nachdem die Neutrophile neutrophile extrazelluläre Fallen freisetzen, als neutrophile extrazelluläre Fallen nachweist, wobei die verbleibenden Partikel von Neutrophilen, nachdem die Neutrophile neutrophile extrazelluläre Fallen freisetzen, eine niedrigere Fluoreszenzintensität haben als die Fluoreszenzintensität, die von den Neutrophilen erhalten wird, die neutrophile extrazelluläre Fallen nicht freigesetzt haben.

11. Probenanalysator (10) gemäß Anspruch 10, wobei der Detektor (200) weiter Streulichtinformationen durch Bestrahlen der Messprobe mit Licht erhält.

12. Probenanalysator (10) gemäß Anspruch 11, wobei die Streulichtinformationen Seitenstreulichtinformationen umfassen.

13. Probenanalysator (10) gemäß Anspruch 11 oder 12, wobei die Informationsverarbeitungseinheit (30) verbleibende Partikel von Neutrophilen, nachdem die Neutrophile neutrophile extrazelluläre Fallen freisetzen, als die neutrophilen extrazellulären Fallen nachweist, wobei die verbleibenden Partikel von Neutrophilen, nachdem die Neutrophile neutrophile extrazelluläre Fallen freisetzen, eine niedrigere Fluoreszenzintensität haben als die Fluoreszenzintensität, die von den Neutrophilen erhalten wird, die neutrophile extrazelluläre Fallen nicht freigesetzt haben, und eine höhere Streulichtintensität als von Lymphozyten erhaltene Streulichtintensität haben.

14. Probenanalysator (10) gemäß irgendeinem der Ansprüche 10 bis 13, wobei das Probenherstellungsteil (100) die Messprobe durch das Mischen mindestens der biologischen Probe und des Nukleinsäure färbenden Fluoreszenzfarbstoffs herstellt, so dass ein osmotischer Druck 245 bis 1680 hPa ist.

15. Probenanalysator (10) gemäß irgendeinem der Ansprüche 10 bis 13, wobei das Probenherstellungsteil (100) die Messprobe durch das Mischen der biologischen Probe, des Nukleinsäure färbenden Fluoreszenzfarbstoffs und eines Tensids herstellt, so dass ein osmotischer Druck 2635 hPa oder niedriger ist.

## Revendications

1. Procédé d'analyse de spécimen destiné à détecter des pièges extracellulaires de neutrophiles, dans lequel le procédé comprend :

la préparation d'un spécimen de mesure à partir d'un spécimen biologique contenant des globules sanguins par coloration, avec une teinture fluorescente de coloration d'acide nucléique, des acides nucléiques contenus dans des neutrophiles n'ayant pas libéré de pièges extracellulaires de neutrophiles ;
l'obtention d'informations optiques incluant des informations de fluorescence par irradiation du spécimen de mesure avec de la lumière ; et
la détection, en tant que pièges extracellulaires de neutrophiles d'après les informations optiques, de particules restantes de neutrophiles après que les neutrophiles libèrent des pièges extracellulaires de neutrophiles, dans lequel les particules restantes de neutrophiles après que les neutrophiles libèrent des pièges extracellulaires de neutrophiles ont une intensité de fluorescence inférieure à l'intensité de fluorescence obtenue à partir des neutrophiles n'ayant pas libéré les pièges extracellulaires de neutrophiles.

**2.** Procédé d'analyse de spécimen selon la revendication 1, dans lequel la préparation du spécimen de mesure comprend l'endommagement de membranes cellulaires des neutrophiles de manière à introduire la teinture fluorescente de coloration d'acide nucléique dans les neutrophiles.

**3.** Procédé d'analyse de spécimen selon la revendication 1 ou 2, dans lequel la préparation du spécimen de mesure comprend, par l'endommagement de membranes cellulaires des neutrophiles de manière à introduire la teinture fluorescente de coloration d'acide nucléique dans les neutrophiles, le fait de rendre l'intensité de fluorescence obtenue à partir des neutrophiles n'ayant pas libéré les pièges extracellulaires de neutrophiles plus élevée que l'intensité de fluorescence obtenue à partir des particules restantes de neutrophiles après que les neutrophiles libèrent des pièges extracellulaires de neutrophiles.

**4.** Procédé d'analyse de spécimen selon l'une quelconque des revendications 1 à 3, dans lequel les informations optiques incluent en outre des informations de lumière diffusée.

**5.** Procédé d'analyse de spécimen selon la revendication 4, dans lequel les informations de lumière diffusée comprennent des informations de lumière diffusée latérale.

**6.** Procédé d'analyse de spécimen selon la revendication 4 ou 5, dans lequel la détection de particules comprend la détection, en tant que pièges extracellulaires de neutrophiles, de particules restantes de neutrophiles après que les neutrophiles libèrent des pièges extracellulaires de neutrophiles, dans lequel les particules restantes de neutrophiles après que les neutrophiles libèrent des pièges extracellulaires de neutrophiles ont une intensité de fluorescence inférieure à l'intensité de fluorescence obtenue à partir des neutrophiles n'ayant pas libéré des pièges extracellulaires de neutrophiles et ont une intensité de lumière diffusée plus élevée que l'intensité de lumière diffusée obtenue à partir de lymphocytes.

**7.** Procédé d'analyse de spécimen selon l'une quelconque des revendications 1 à 6, dans lequel l'obtention des informations optiques comprend l'obtention des informations optiques par un cytomètre en flux.

**8.** Procédé d'analyse de spécimen selon l'une quelconque des revendications 1 à 7, dans lequel la préparation du spécimen de mesure comprend le mélange d'au moins le spécimen biologique et de la teinture fluorescente de coloration d'acide nucléique de sorte qu'une pression osmotique soit de 245 hPa à 1680 hPa.

**9.** Procédé d'analyse de spécimen selon l'une quelconque des revendications 1 à 7, dans lequel la préparation du spécimen de mesure comprend le mélange d'au moins le spécimen biologique, de la teinture fluorescente de coloration d'acide nucléique, et d'un tensioactif de sorte qu'une pression osmotique soit de 2635 hPa ou moins.

**10.** Analyseur de spécimen (10) destiné à détecter des pièges extracellulaires de neutrophiles, dans lequel l'analyseur de spécimen (10) comprend :

une partie de préparation de spécimen (100) qui prépare un spécimen de mesure à partir d'un spécimen biologique contenant des globules sanguins par coloration, avec une teinture fluorescente de coloration d'acide nucléique, d'acides nucléiques contenus dans des neutrophiles n'ayant pas libéré de pièges extracellulaires de neutrophiles ;
un détecteur (200) qui obtient des informations de fluorescence par irradiation du spécimen de mesure avec de la lumière ; et
une unité de traitement d'informations (30) qui détecte, en tant que pièges extracellulaires de neutrophiles, des particules restantes de neutrophiles après que les neutrophiles libèrent des pièges extracellulaires de neutrophiles, dans lequel les particules restantes de neutrophiles après que les neutrophiles libèrent des pièges extracellulaires de neutrophiles ont une intensité de fluorescence inférieure à l'intensité de fluorescence obtenue à partir des neutrophiles n'ayant pas libéré des pièges extracellulaires de neutrophiles.

**11.** Analyseur de spécimen (10) selon la revendication 10, dans lequel le détecteur (200) obtient en outre des informations de lumière diffusée par irradiation du spécimen de mesure avec de la lumière.

**12.** Analyseur de spécimen (10) selon la revendication 11, dans lequel les informations de lumière diffusée comprennent des informations de lumière diffusée latérale.

**13.** Analyseur de spécimen (10) selon la revendication 11 ou 12, dans lequel l'unité de traitement d'informations (30)

détecte, en tant que pièges extracellulaires de neutrophiles, des particules restantes de neutrophiles après que les neutrophiles libèrent des pièges extracellulaires de neutrophiles, dans lequel les particules restantes de neutrophiles après que les neutrophiles libèrent des pièges extracellulaires de neutrophiles ont une intensité de fluorescence inférieure à l'intensité de fluorescence obtenue à partir des neutrophiles n'ayant pas libéré de pièges extracellulaires de neutrophiles et ont une intensité de lumière diffusée plus élevée que l'intensité de lumière diffusée obtenue à partir de lymphocytes.

**14.** Analyseur de spécimen (10) selon l'une quelconque des revendications 10 à 13, dans lequel la partie de préparation de spécimen (100) prépare le spécimen de mesure par mélange d'au moins le spécimen biologique et de la teinture fluorescente de coloration d'acide nucléique de sorte qu'une pression osmotique soit de 245 à 1680 hPa.

**15.** Analyseur de spécimen (10) selon l'une quelconque des revendications 10 à 13, dans lequel la partie de préparation de spécimen (100) prépare le spécimen de mesure par mélange du spécimen biologique, de la teinture fluorescente de coloration d'acide nucléique, et d'un tensioactif de sorte qu'une pression osmotique soit de 2635 hPa ou moins.

Fig. 1

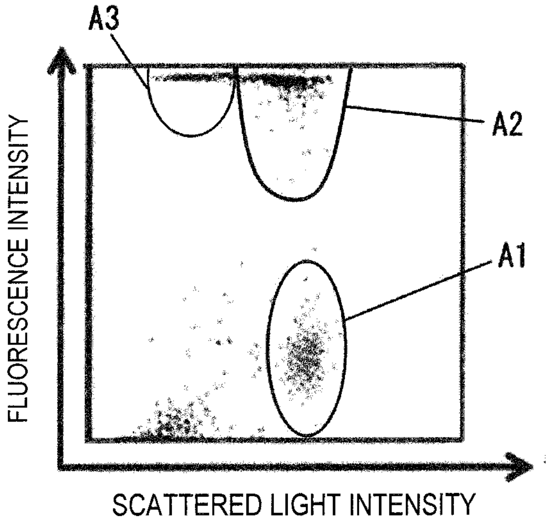
A3
A2
A1
FLUORESCENCE INTENSITY
SCATTERED LIGHT INTENSITY

Fig. 2

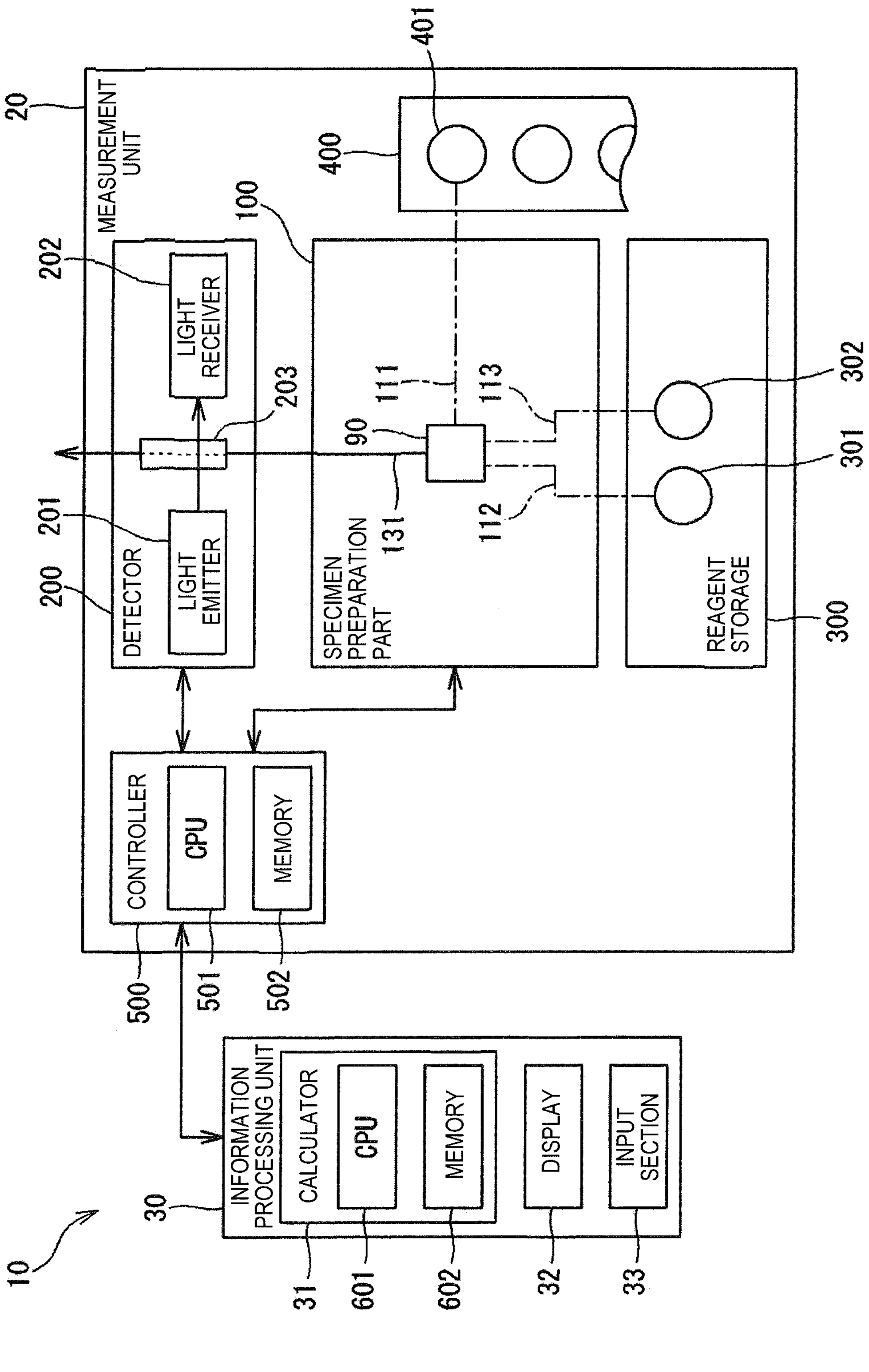

10
20
MEASUREMENT UNIT
200
DETECTOR
201
LIGHT EMITTER
202
LIGHT RECEIVER
203
100
SPECIMEN PREPARATION PART
90
111
112
113
131
400
401
300
REAGENT STORAGE
301
302
500
CONTROLLER
501
CPU
502
MEMORY
30
INFORMATION PROCESSING UNIT
31
CALCULATOR
601
CPU
602
MEMORY
32
DISPLAY
33
INPUT SECTION

Fig. 3

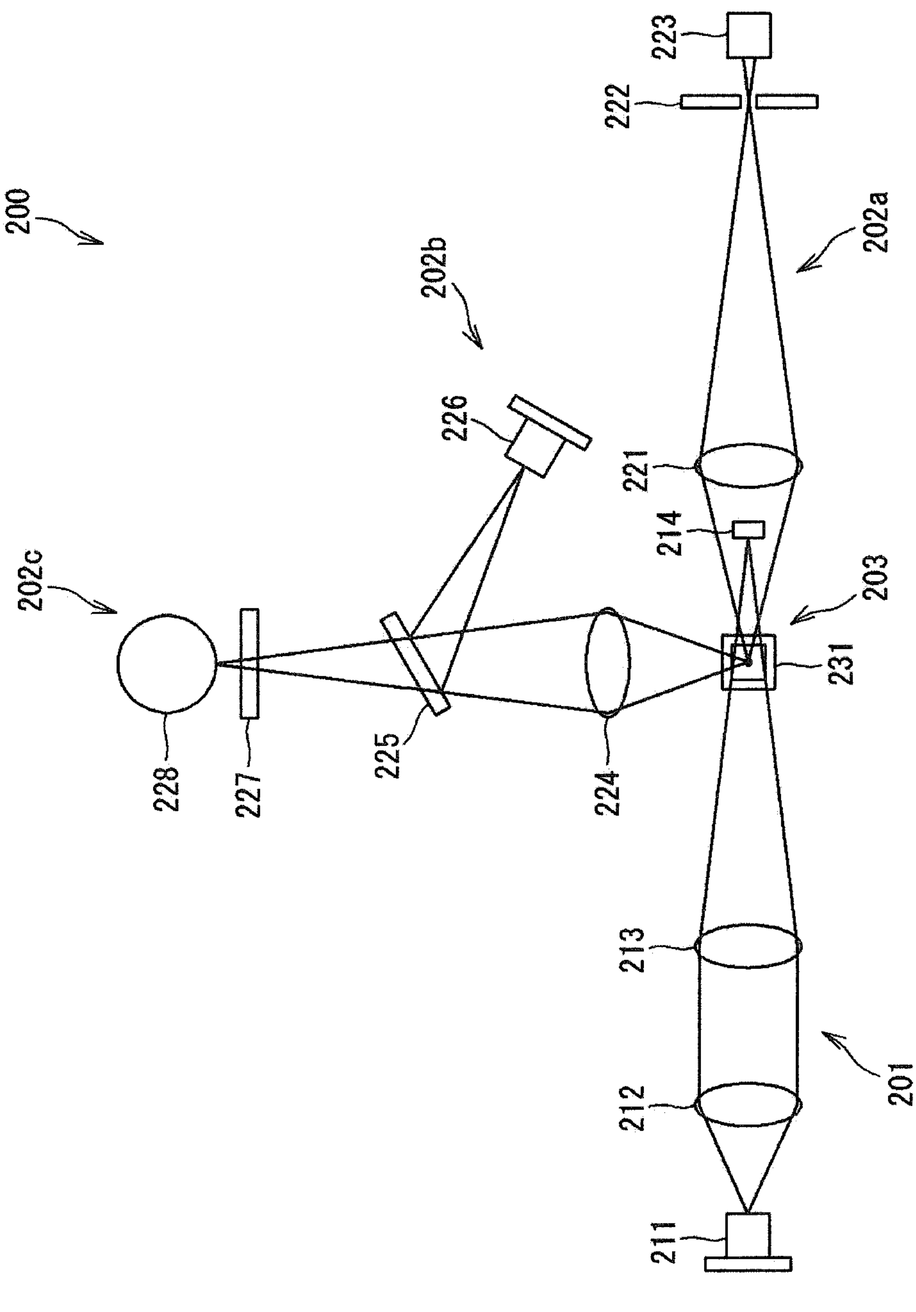
200
202b
202c
202a
226
223
222
221
214
203
231
228
227
225
224
213
212
211
201

Fig. 4

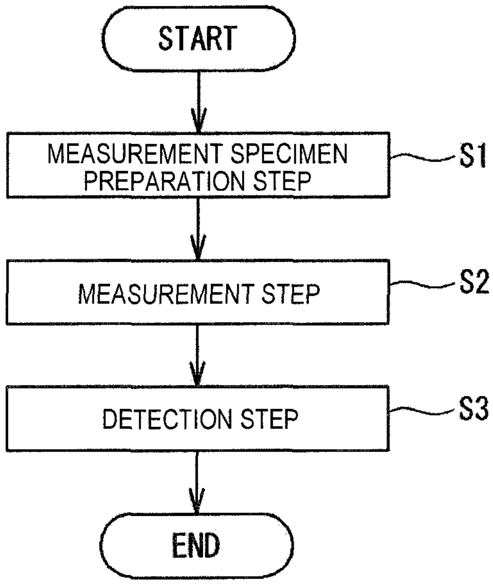
START
MEASUREMENT SPECIMEN PREPARATION STEP
S1
MEASUREMENT STEP
S2
DETECTION STEP
S3
END

Fig. 5

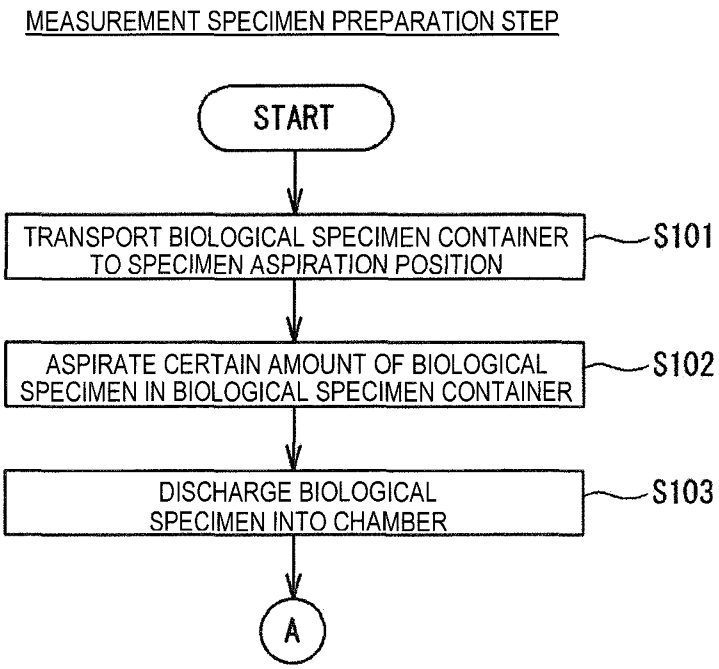
MEASUREMENT SPECIMEN PREPARATION STEP
START
TRANSPORT BIOLOGICAL SPECIMEN CONTAINER TO SPECIMEN ASPIRATION POSITION
S101
ASPIRATE CERTAIN AMOUNT OF BIOLOGICAL SPECIMEN IN BIOLOGICAL SPECIMEN CONTAINER
S102
DISCHARGE BIOLOGICAL SPECIMEN INTO CHAMBER
S103
A

Fig. 6

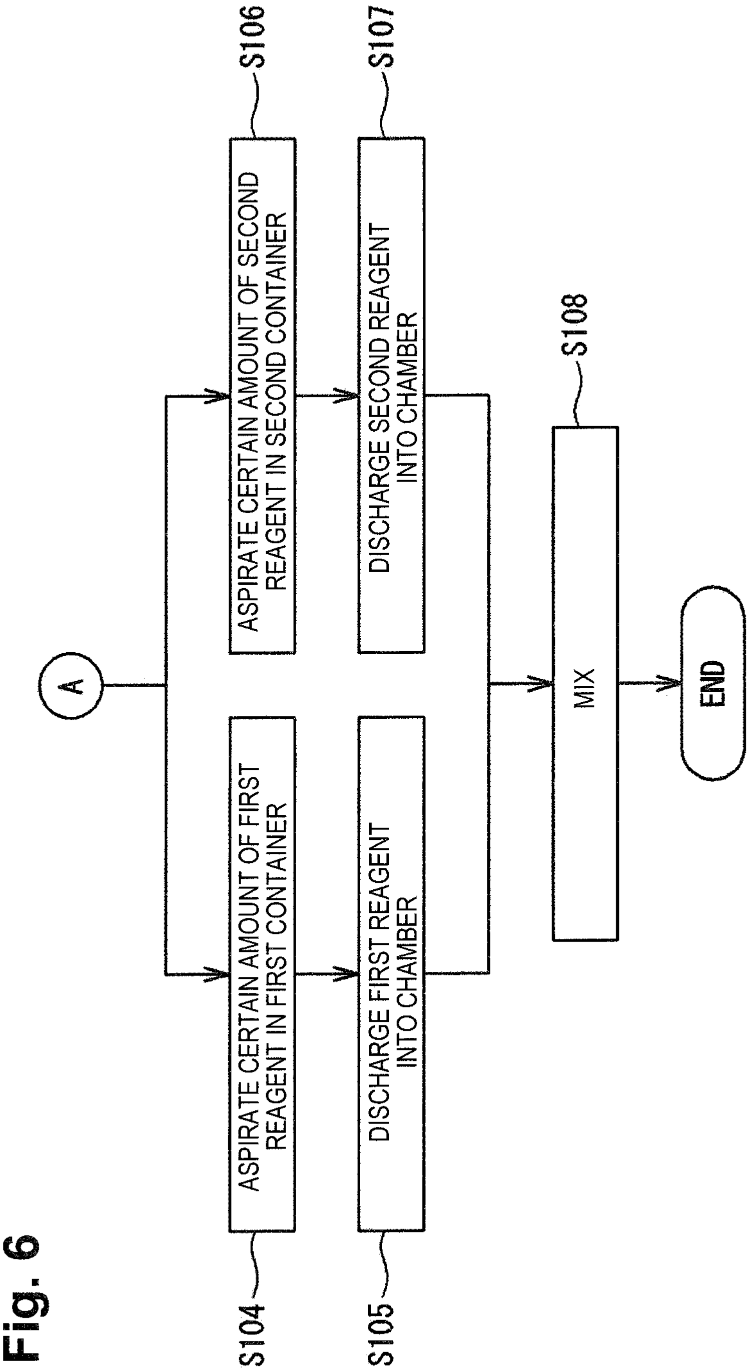
A
ASPIRATE CERTAIN AMOUNT OF FIRST REAGENT IN FIRST CONTAINER
S104
DISCHARGE FIRST REAGENT INTO CHAMBER
S105
ASPIRATE CERTAIN AMOUNT OF SECOND REAGENT IN SECOND CONTAINER
S106
DISCHARGE SECOND REAGENT INTO CHAMBER
S107
MIX
S108
END

Fig. 7

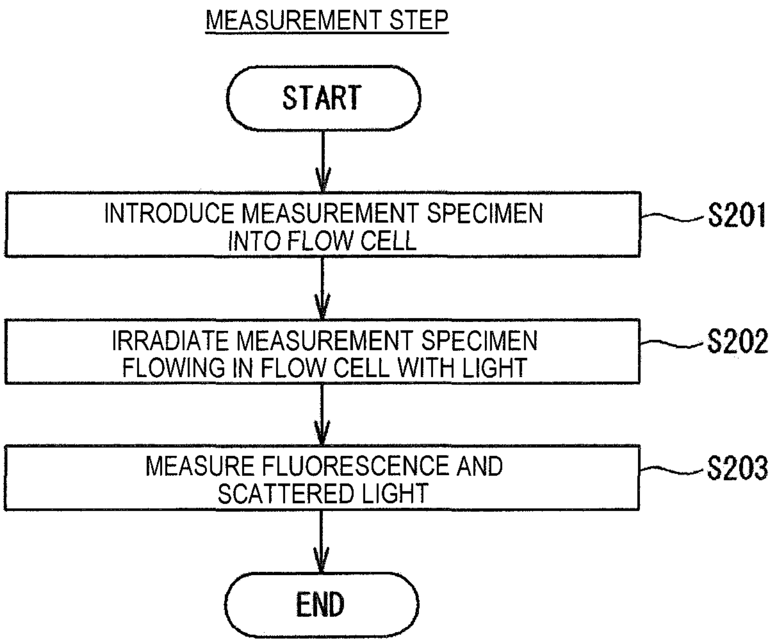
MEASUREMENT STEP
START
INTRODUCE MEASUREMENT SPECIMEN INTO FLOW CELL
S201
IRRADIATE MEASUREMENT SPECIMEN FLOWING IN FLOW CELL WITH LIGHT
S202
MEASURE FLUORESCENCE AND SCATTERED LIGHT
S203
END

Fig. 8

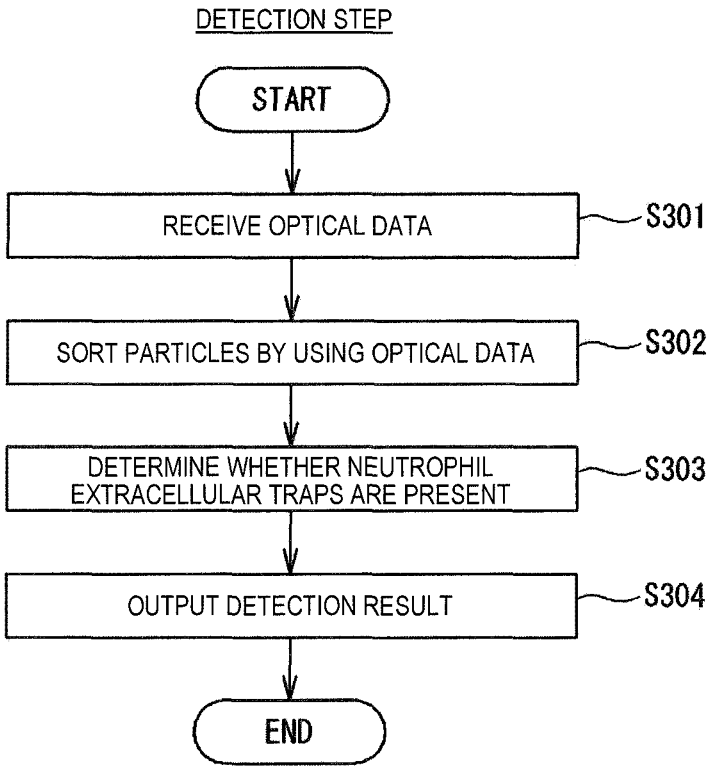
DETECTION STEP
START
RECEIVE OPTICAL DATA
S301
SORT PARTICLES BY USING OPTICAL DATA
S302
DETERMINE WHETHER NEUTROPHIL EXTRACELLULAR TRAPS ARE PRESENT
S303
OUTPUT DETECTION RESULT
S304
END

Fig. 9

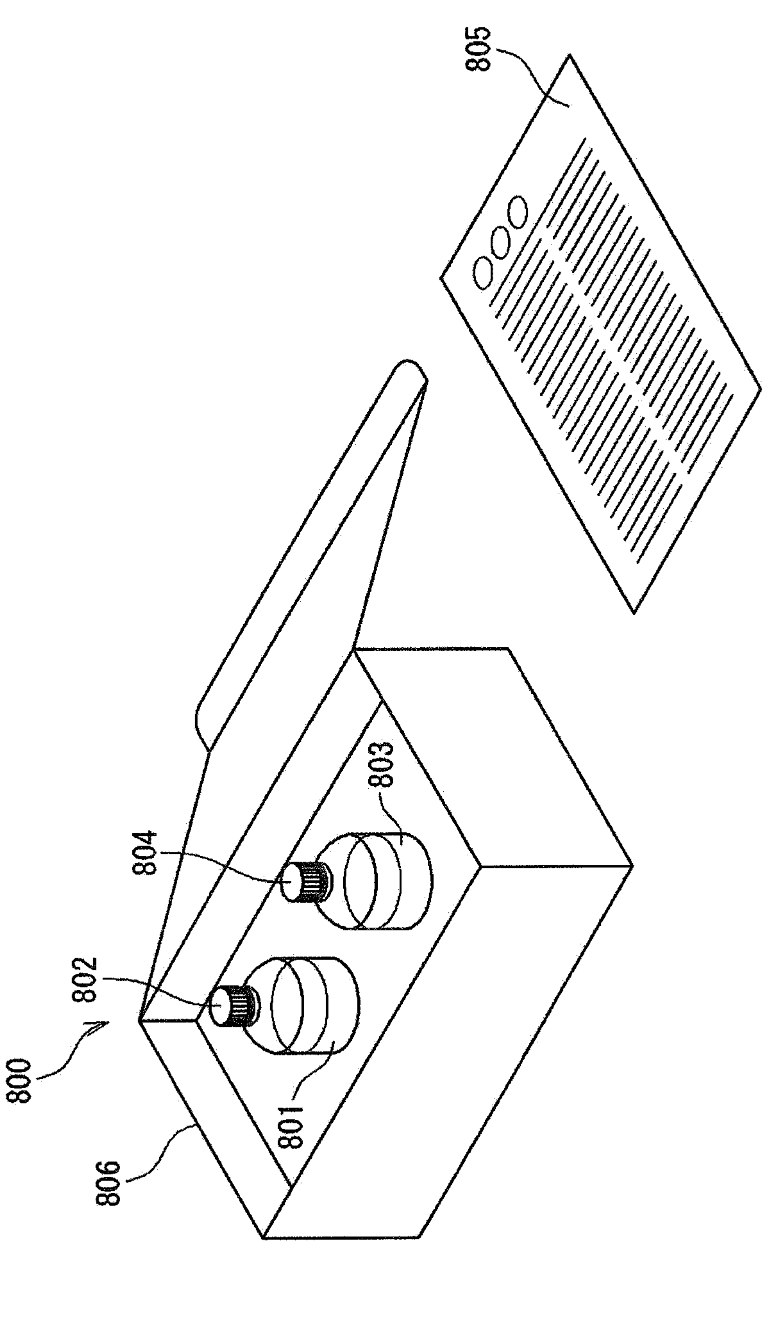

Fig. 10

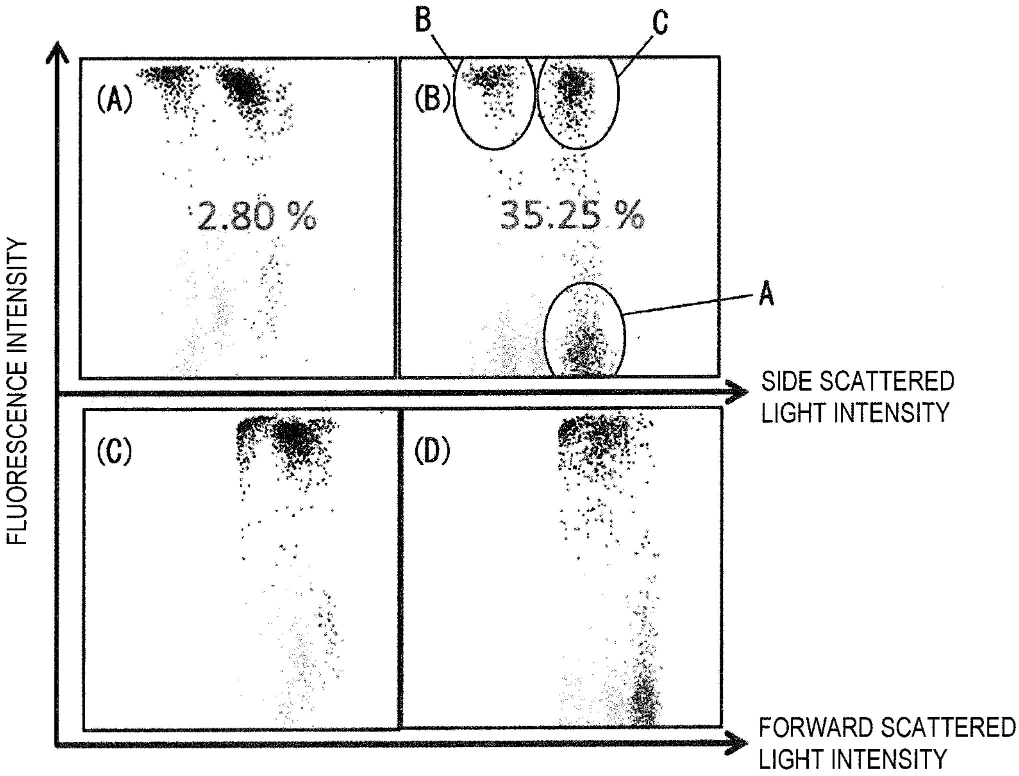
B
C
(A)
(B)
2.80 %
35.25 %
A
FLUORESCENCE INTENSITY
SIDE SCATTERED LIGHT INTENSITY
(C)
(D)
FORWARD SCATTERED LIGHT INTENSITY

Fig. 11

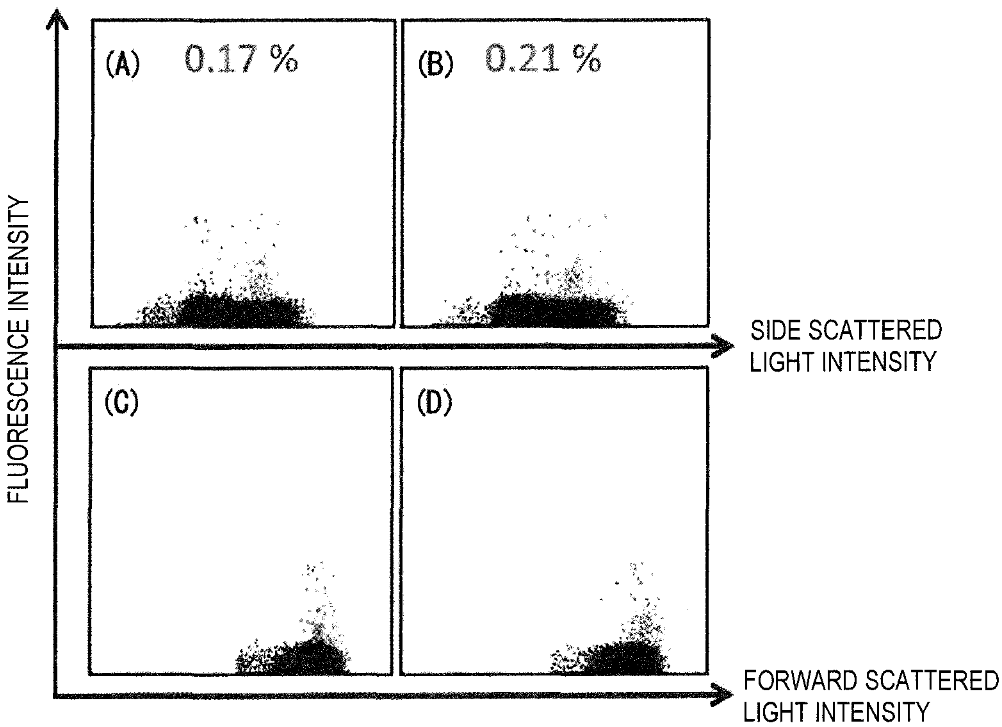
(A) 0.17 %
(B) 0.21 %
(C)
(D)
FLUORESCENCE INTENSITY
SIDE SCATTERED LIGHT INTENSITY
FORWARD SCATTERED LIGHT INTENSITY

Fig. 12

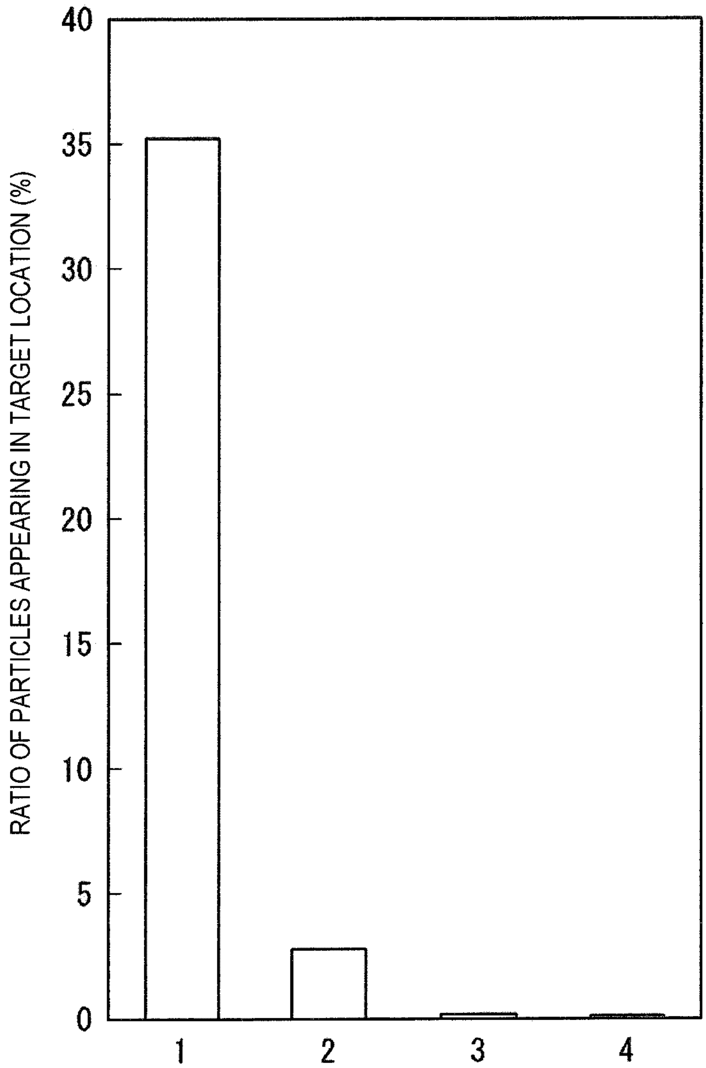
RATIO OF PARTICLES APPEARING IN TARGET LOCATION (%)
40
35
30
25
20
15
10
5
0
1
2
3
4

Fig. 13

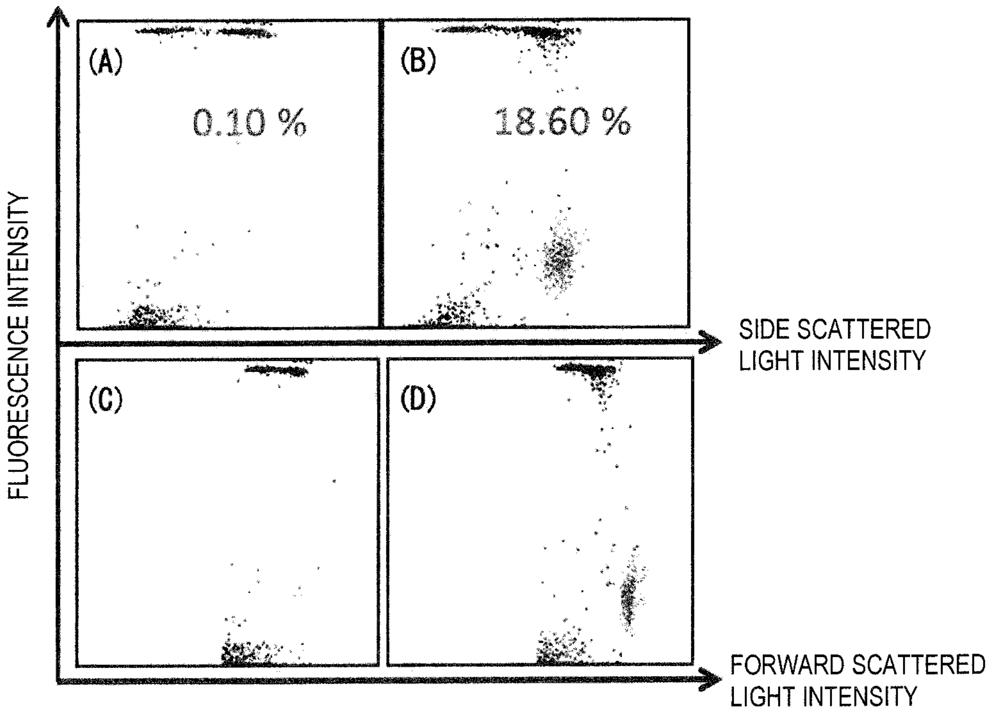
(A)
0.10 %
(B)
18.60 %
(C)
(D)
FLUORESCENCE INTENSITY
SIDE SCATTERED LIGHT INTENSITY
FORWARD SCATTERED LIGHT INTENSITY

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2587262 A2


### Non-patent literature cited in the description


- **WALID M. AL-GHOUL et al.** Evidence for simvastatin anti-inflammatory actions based on quantitative analyses of NETosis and other inflammation/oxidation markers. *Results in Immunology,* 2014, vol. 4, 14-22
- **BRINKMANN et al.** *Front Immunol,* 2013, vol. 3, 413